(19) Europäisches Patentamt
European Patent Office
Office européen des brevets

(11) EP 4 782 433 A1

(12) EUROPEAN PATENT APPLICATION
published in accordance with Art. 153(4) EPC

(43) Date of publication:
**29.07.2026 Bulletin 2026/31**

(21) Application number: **24868352.6**

(22) Date of filing: **20.09.2024**

(51) International Patent Classification (IPC):
***C07D 213/76*** *(2006.01)* ***A61K 31/44*** *(2006.01)*
***A61P 35/00*** *(2006.01)*

(52) Cooperative Patent Classification (CPC):
**A61K 31/44; A61P 35/00; C07D 213/76**

(86) International application number:
**PCT/JP2024/033634**

(87) International publication number:
**WO 2025/063275 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.09.2023 JP 2023156465**

(71) Applicant: **CHUGAI SEIYAKU KABUSHIKI KAISHA**
**Tokyo 115-8543 (JP)**

(72) Inventors:
- **IDE Takafumi**
  **Tokyo 115-8543 (JP)**
- **HOSONO Shu**
  **Tokyo 115-8543 (JP)**
- **KUWATA Kazuaki**
  **Tokyo 115-8543 (JP)**

(74) Representative: **Vossius & Partner**
**Patentanwälte Rechtsanwälte mbB**
**Siebertstraße 3**
**81675 München (DE)**

# (54) METHOD FOR PRODUCING ARYLAMIDE DERIVATIVE

(57) The present disclosure provides, for example, a method for producing a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), the method comprising mixing 2-(4-cyclopropyl-2-fluoroanilino)-3,4-difluoro-5-[[3-fluoro-2-(methylsulfamoylamino)pyridin-4-yl]methyl]benzamide, sodium tert-butoxide, and m-cresol in a solvent to obtain a mixture containing compound 3. According to the present disclosure, there is provided a method for producing compound 3 that makes it possible to obtain a product with a higher content of compound 3.

EP 4 782 433 A1

## Description

### Technical Field

The present disclosure relates to a method for producing an aryl amide derivative.

### Background Art

A sodium salt (in the present disclosure, also referred to as "compound 3") of 2-(4-cyclopropyl-2-fluoroanilino)-3,4-difluoro-5-[[3-fluoro-2-(methylsulfamoylamino)pyridin-4-yl]methyl]benzamide (in the present disclosure, also referred to as "compound 1" or "compound 1A") is known to be useful in the treatment or prevention of cancer (see patent document 1).

As for the method for producing compound 3 and its production intermediate, compound 1, the method described in Synthetic Example 4 below is known (see patent document 2).

### Citation List

### Patent Literature

Patent document 1: WO 2021/149776
Patent document 2: WO 2023/003014

## Summary of Invention

### Technical Problem

The method described in Synthetic Example 4 below was found to have room for improvement in the content of compound 3 in the resulting product. In addition, the method for producing compound 1 described in Synthetic Example 4 below was found to have room for improvement in simplicity.

It is an object of the present disclosure to provide a method for producing compound 3 that makes it possible to obtain a product with a higher content of compound 3. It is another object of the present disclosure to provide a method for producing compound 1 that makes it possible to more simply obtain a product with a high content of compound 1.

### Solution to Problem

The present disclosure includes the methods according to A1 to A45 below.

A1:
A method for producing a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), the method comprising step (A-1) below:

(A-1) mixing (a), (b), and (c) below in a first solvent to obtain a mixture containing compound 3:

(a) 2-(4-cyclopropyl-2-fluoroanilino)-3,4-difluoro-5-[[3-fluoro-2-(methylsulfamoylamino)pyridin-4-yl]methyl]benzamide (compound 1);
(b) a sodium base; and
(c) at least one organic acid selected from the group consisting of compounds represented by general formula (A) below:

[Chemical Formula 1]

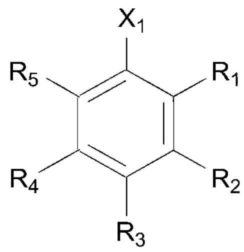

(A)

wherein

$X_1$ is a hydroxy group or a mercapto group; and
$R_1$, $R_2$, $R_3$, $R_4$, and $R_5$ are each independently a hydrogen atom, a halogen atom, a C1-6 alkyl group, a hydroxy group, a C1-6 alkoxy group, a phenoxy group, a nitro group, or a cyano group.

A2:
The method according to A1, wherein the sodium base comprises at least one selected from the group consisting of sodium C1-6 alkoxides, sodium hydroxide, sodium hydride, and sodium hexamethyldisilazide.
A3:
The method according to A1 or A2, wherein the sodium base comprises at least one selected from the group consisting of sodium tert-butoxide, sodium tert-pentoxide, sodium hydroxide, sodium hydride, sodium methoxide, sodium ethoxide, and sodium hexamethyldisilazide.
A4:
The method according to any of A1 to A3, wherein the sodium base comprises at least one selected from the group consisting of sodium tert-butoxide, sodium tert-pentoxide, and sodium hydroxide.
A5:
The method according to any of A1 to A4, wherein the sodium base is sodium tert-butoxide.
A6: The method according to any of A1 to A5, wherein

$X_1$ is a hydroxy group, and
$R_1$, $R_2$, $R_3$, $R_4$, and $R_5$ are each independently a hydrogen atom or a C1-6 alkyl group.

A7:
The method according to any of A1 to A6, wherein said organic acid is at least one selected from the group consisting of o-cresol, m-cresol, p-cresol, phenol, and dibutylhydroxytoluene.
A8:
The method according to any of A1 to A7, wherein said organic acid is at least one selected from the group consisting of m-cresol, phenol, and dibutylhydroxytoluene.
A9:
The method according to any of A1 to A8, wherein said organic acid is at least one selected from the group consisting of m-cresol and phenol.
A10:
The method according to any of A1 to A9, wherein said organic acid is m-cresol.
A11:
The method according to any of A1 to A10, wherein said organic acid is an acid weaker than compound 1.
A12:
The method according to any of A1 to A11, wherein said organic acid has a pKa of 7.0 to 12.0 in water at 25°C.
A13:
The method according to any of A1 to A12, wherein said organic acid has a pKa of 7.0 to 11.0 in water at 25°C.
A14:
The method according to any of A1 to A13, wherein said organic acid has a pKa of 8.0 to 11.0 in water at 25°C.
A15:
The method according to any of A1 to A14, wherein a solubility at 25°C of a sodium salt of said organic acid in a mixed solvent of tetrahydrofuran, acetone, tert-butyl methyl ether, and water (a volume ratio of tetrahydrofuran, acetone, and

tert-butyl methyl ether is 8:10:5, and a content of water is 3% by mass with respect to a mass of said mixed solvent) is 3 mmol/L or more.

A16:

The method according to any of A1 to A15, wherein a solubility at 25°C of a sodium salt of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 0.05 mol/L or more.

A17:

The method according to any of A1 to A16, wherein a solubility at 25°C of a sodium salt of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 0.08 mol/L or more.

A18:

The method according to any of A1 to A17, wherein a solubility at 25°C of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 0.05 mol/L or more.

A19:

The method according to any of A1 to A18, wherein a solubility at 25°C of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 0.08 mol/L or more.

A20:

The method according to any of A1 to A19, wherein a solubility at 25°C of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 0.10 mol/L or more.

A21:

The method according to any of A1 to A20, wherein a molar equivalent of the organic acid mixed in step (A-1) is 0.1 to 3 with respect to the sodium base.

A22:

The method according to any of A1 to A21, wherein a molar equivalent of the organic acid mixed in step (A-1) is 0.1 to 1.5 with respect to the sodium base.

A23:

The method according to any of A1 to A22, wherein a molar equivalent of the organic acid mixed in step (A-1) is 0.3 to 0.6 with respect to the sodium base.

A24:

The method according to any of A1 to A23, wherein a molar equivalent of the sodium base mixed in step (A-1) is 0.3 to 3 with respect to compound 1.

A25:

The method according to any of A1 to A24, wherein a molar equivalent of the sodium base mixed in step (A-1) is 0.5 to 1.5 with respect to compound 1.

A26:

The method according to any of A1 to A25, wherein a molar equivalent of the sodium base mixed in step (A-1) is 0.8 to 1.2 with respect to compound 1.

A27:

The method according to any of A1 to A26, wherein the first solvent comprises at least one selected from the group consisting of ether solvents, ketone solvents, and amide solvents.

A28:

The method according to any of A1 to A27, wherein the first solvent comprises at least one selected from the group consisting of tetrahydrofuran, acetone, and 1,3-dimethyl-2-imidazolidinone.

A29:

The method according to any of A1 to A28, wherein the first solvent comprises at least one selected from the group consisting of tetrahydrofuran and acetone.

A30:

The method according to any of A1 to A29, wherein a temperature of the first solvent in step (A-1) is -25°C to 40°C.

A31:

The method according to any of A1 to A30, wherein a temperature of the first solvent in step (A-1) is -10°C to 10°C.

A32:
The method according to any of A1 to A31, wherein a volume of the first solvent in step (A-1) is 6 to 16 mL per 1 g of a mass of compound 3 produced in step (A-1).
A33:
The method according to any of A1 to A32, wherein a volume of the first solvent in step (A-1) is 8 to 14 mL per 1 g of a mass of compound 3 produced in step (A-1).
A34:
The method according to any of A1 to A33, wherein a volume of the first solvent in step (A-1) is 10 to 12 mL per 1 g of a mass of compound 3 produced in step (A-1).
A35:
The method according to any of A1 to A34, further comprising step (A-2) below:
(A-2) mixing the mixture obtained in step (A-1) with a second solvent.
A36:
The method according to A35, wherein the second solvent comprises at least one selected from the group consisting of ether solvents, ketone solvents, and hydrocarbon solvents.
A37:
The method according to A35 or A36, wherein the second solvent comprises at least one selected from the group consisting of acetone, methyl ethyl ketone, tert-butyl methyl ether, cyclopentyl methyl ether, and n-heptane.
A38:
The method according to any of A35 to A37, wherein the second solvent comprises at least one selected from the group consisting of acetone and tert-butyl methyl ether.
A39:
The method according to any of A35 to A38, wherein a temperature of the second solvent in step (A-2) is -25°C to 50°C.
A40:
The method according to any of A35 to A39, wherein a temperature of the second solvent in step (A-2) is 5°C to 40°C.
A41:

The method according to any of A35 to A40, wherein a volume of the second solvent in step (A-2) is 0.3 to 3 times a volume of the first solvent.
A42:
The method according to any of A35 to A41, wherein a volume of the second solvent in step (A-2) is 0.5 to 2 times a volume of the first solvent.
A43:
The method according to any of A35 to A42, wherein a volume of the second solvent in step (A-2) is 0.7 to 1.3 times a volume of the first solvent.
A44:
The method according to any of A1 to A43, further comprising step (A-3) below:
(A-3) obtaining compound 3 from the mixture obtained in step (A-1) or (A-2).
A45: The method according to A44, wherein in step (A-3), compound 3 is obtained from the mixture obtained in step (A-2) by solid-liquid separation.

In the methods described in A1 to A45 above, the organic acid of general formula (A) is used when making compound 1 into the sodium salt (compound 3). Compared to the method described in Synthetic Example 4 below, where such an organic acid is not used, the methods described in A1 to A45 above make it possible to obtain a product with a higher content of compound 3.

The present disclosure also includes the methods according to B1 to B16 below.

B1:
A method for producing 2-(4-cyclopropyl-2-fluoroanilino)-3,4-difluoro-5-[[3-fluoro-2-(methylsulfamoylamino)pyridin-4-yl]methyl]benzamide (compound 1), the method comprising steps (B-1), (B-2), and (B-3) below:

(B-1) mixing (d), (e), and (f) below to obtain a mixture containing compound 1:

(d) 5-[(2-amino-3-fluoropyridin-4-yl)methyl]-2-(4-cyclopropyl-2-fluoroanilino)-3,4-difluorobenzamide (compound 2);
(e) N-methylsulfamoyl chloride; and
(f) an aqueous solution of an inorganic salt;

(B-2) removing an aqueous layer from the mixture obtained in step (B-1) by a separating operation; and
(B-3) mixing the mixture obtained in step (B-2) with a fourth solvent to remove said inorganic salt.

B2:
The method according to B1, wherein the aqueous solution of an inorganic salt is at least one selected from the group consisting of an aqueous sodium chloride solution, an aqueous ammonium chloride solution, an aqueous sodium dihydrogen phosphate solution, and an aqueous potassium dihydrogen phosphate solution.
B3:
The method according to B1 or B2, wherein the aqueous solution of an inorganic salt is at least one selected from the group consisting of an aqueous sodium chloride solution and an aqueous ammonium chloride solution.
B4:
The method according to any of B1 to B3, wherein the aqueous solution of an inorganic salt is an aqueous sodium chloride solution.
B5:
The method according to any of B1 to B4, wherein step (B-1) is carried out in a third solvent.
B6:
The method according to B5, wherein the third solvent comprises at least one selected from the group consisting of ether solvents, ketone solvents, and ester solvents.
B7:
The method according to B5 or B6, wherein the third solvent comprises at least one selected from the group consisting of tetrahydrofuran, 2-methyltetrahydrofuran, 4-methyltetrahydropyran, acetone, methyl ethyl ketone, ethyl acetate, and isopropyl acetate.
B8:
The method according to any of B5 to B7, wherein the third solvent is tetrahydrofuran.
B9:
The method according to any of B5 to B8, wherein a temperature of the third solvent in step (B-1) is -20°C to 10°C.
B10:
The method according to any of B5 to B9, wherein a temperature of the third solvent in step (B-1) is -20°C to 5°C.
B11:
The method according to any of B1 to B10, wherein the fourth solvent comprises at least one selected from the group consisting of ketone solvents, ether solvents, ester solvents, hydrocarbon solvents, aromatic solvents, alcohol solvents, and halogen solvents.
B12:
The method according to any of B1 to B11, wherein the fourth solvent comprises at least one selected from the group consisting of ketone solvents and ether solvents.
B13:
The method according to any of B1 to B12, wherein the fourth solvent is a solvent that does not dissolve sodium chloride and dissolves compound 1.
B14:
The method according to any of B1 to B13, wherein the fourth solvent comprises at least one selected from the group consisting of acetone, methyl ethyl ketone, and tetrahydrofuran.
B15:
The method according to any of B1 to B14, wherein a temperature of the fourth solvent in step (B-3) is -20°C to 25°C.
B16:
The method according to any of B1 to B15, wherein a temperature of the fourth solvent in step (B-3) is -10°C to 15°C.

In the methods described in B1 to B16 above, there is no need to carry out concentration in order to remove the inorganic salt from the mixture containing compound 1. Compared to the method described in Synthetic Example 4 (4-1) below, where concentration is carried out in order to remove the inorganic salt, the methods described in B1 to B16 above make it possible to more simply obtain a product with a high content of compound 1.
In addition, in the methods described in B1 to B16 above, there is no need to carry out precipitation of compound 1. Compared to the method described in Synthetic Example 4 (4-2) below, where precipitation of compound 1 is carried out, the methods described in B1 to B16 above make it possible to more simply obtain a product with a high content of compound 1.
The present disclosure also includes the methods according to C1 to C3 below.

C1:
A method for producing a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), the method comprising step (C-1) below:
(C-1) producing 2-(4-cyclopropyl-2-fluoroanilino)-3,4-difluoro-5-[[3-fluoro-2-(methylsulfamoylamino)pyridin-4-yl]methyl]benzamide (compound 1) by the method according to any of B1 to B16.
C2:
The method according to C1, further comprising:
producing compound 3 from compound 1 obtained in step (C-1), using a sodium base.
C3:
A method for producing a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), the method comprising steps (C-1) and (C-2) below:

(C-1) producing 2-(4-cyclopropyl-2-fluoroanilino)-3,4-difluoro-5-[[3-fluoro-2-(methylsulfamoylamino)pyridin-4-yl]methyl]benzamide (compound 1) by the method according to any of B1 to B16; and
(C-2) producing compound 3 by the method according to any of A1 to A45, using compound 1 obtained in step (C-1).

The present disclosure also includes the compositions according to D1 to D7 or the method below.

D1:
A composition comprising a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), wherein a content of compound 3 is 98.0% by mass or more with respect to a mass of said composition.
D2:
The composition according to D1, wherein a content of compound 3 is 99.0% by mass or more with respect to a mass of said composition.
D3:
The composition according to D1 or D2, wherein a peak area of compound 3 obtained by high-performance liquid chromatography (HPLC) analysis at 278 nm for said composition is 99.0% or more with respect to a sum of peak areas of all substances detected in said HPLC analysis.
D4:
A composition comprising a sodium salt of 2-(4-cyclopropyl-2-fluoroanilino)-3,4-difluoro-5-[[3-fluoro-2-(methylsulfamoylamino)pyridin-4-yl]methyl]benzamide (compound 3) and 2-(4-cyclopropyl-2-fluoroanilino)-3,4-difluoro-5-[[3-fluoro-2-(methylsulfamoylamino)pyridin-4-yl]methyl]benzoic acid (compound 4),
wherein a peak area of compound 4 obtained by high-performance liquid chromatography (HPLC) analysis at 278 nm for said composition is 0.20% or less with respect to a peak area of compound 3 obtained by said HPLC analysis.
D5:
A composition, wherein a peak area of compound 4 obtained by high-performance liquid chromatography (HPLC) analysis at 278 nm for said composition is 0.05% or less with respect to a peak area of compound 3 obtained by said HPLC analysis.
D6:
The composition according to D4 or D5, which is a pharmaceutical composition.
D7:
The composition according to D6, which is a pharmaceutical composition for treatment or prevention of cancer.
D8:
The method according to any of A1 to A45 and C1 to C3, which is a method for producing the composition according to any of D1 to D7.

The present disclosure also includes the method according to E1 below.
E1:
The method according to any of A1 to A45 and C1 to C3, wherein a product with a high content of compound 3 can be obtained.

Advantageous Effects of Invention

According to the present disclosure, there is provided a method for producing a specific aryl amide derivative that is useful for the treatment or prevention of cancer, the method making it possible to obtain a product with a higher content of the aryl amide derivative. There is also provided a method for producing a production intermediate for such an aryl amide derivative, the method making it possible to more simply obtain a product with a high content of the production intermediate.

Brief Description of Drawings

[Fig. 1] Fig. 1 shows a powder X-ray diffraction pattern of sample 1a (Form I).
[Fig. 2] Fig. 2 shows a powder X-ray diffraction pattern of sample 1b (Form I).
[Fig. 3] Fig. 3 shows a powder X-ray diffraction pattern of sample 1c.

Description of Embodiments

Exemplary embodiments of the present invention are described below.
As used in the present disclosure, when a numerical range is indicated using "to", that numerical range includes the values at both ends of the range. For example, "having a pKa of 7.0 to 12.0" means that the pKa is 7.0 or more and 12.0 or less.
As used in the present disclosure, "halogen atom" refers to a fluorine atom, a chlorine atom, a bromine atom, or an iodine atom.
As used in the present disclosure, "C1-6 alkyl group" means a straight- or branched-chain alkyl group of 1 to 6 carbon atoms. Examples include methyl, ethyl, *n*-propyl, isopropyl, *n*-butyl, *sec*-butyl, *tert*-butyl, 1-methylpropyl, *n*-pentyl, 1-methylbutyl, 2-methylbutyl, 3-methylbutyl, 1,1-dimethylpropyl, 2,2-dimethylpropyl, 1,2-dimethylpropyl, 1-ethylpropyl, *n*-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl groups.
As used in the present disclosure, "C1-6 alkoxy group" means an alkoxy group having a straight- or branched-chain alkyl group of 1 to 6 carbon atoms. Examples include methoxy, ethoxy, n-propoxy, isopropoxy, n-butoxy, sec-butoxy, tert-butoxy, n-pentoxy, tert-pentoxy, and n-hexoxy groups.
As used in the present disclosure, "sodium C1-6 alkoxide" means a sodium alkoxide having a straight- or branched-chain alkyl group of 1 to 6 carbon atoms. Examples include sodium methoxide, sodium ethoxide, sodium n-propoxide, sodium isopropoxide, sodium n-butoxide, sodium sec-butoxide, sodium tert-butoxide, sodium n-pentoxide, sodium tert-pentoxide, and sodium n-hexoxide.
A first aspect of the present disclosure provides a method for producing a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), the method comprising step (A-1) below:
(A-1) mixing (a), (b), and (c) below in a first solvent to obtain a mixture containing compound 3:

(a) 2-(4-cyclopropyl-2-fluoroanilino)-3,4-difluoro-5-[[3-fluoro-2-(methylsulfamoylamino)pyridin-4-yl]methyl]benzamide (compound 1);
(b) a sodium base; and
(c) at least one organic acid selected from the group consisting of compounds represented by general formula (A) below:

[Chemical Formula 2]

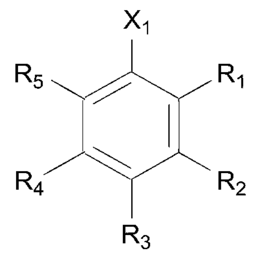

(A)

wherein

$X_1$ is a hydroxy group or a mercapto group; and
$R_1$, $R_2$, $R_3$, $R_4$, and $R_5$ are each independently a hydrogen atom, a halogen atom, a C1-6 alkyl group, a hydroxy group, a C1-6 alkoxy group, a phenoxy group, a nitro group, or a cyano group.

Compound 3 is represented by the formula below.

[Chemical Formula 3]

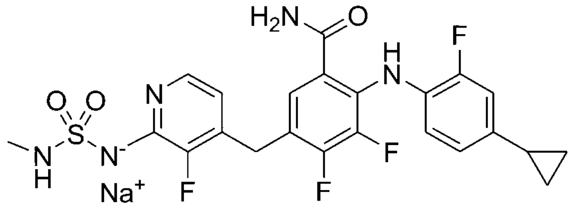

3

Compound 1 (in the present disclosure, also referred to as "compound 1A") is represented by the formula below.

[Chemical Formula 4]

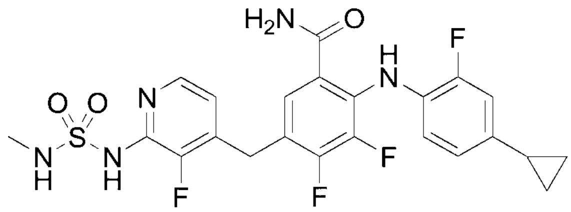

1

Compound 1 can be produced in accordance with, for example, the method of the second aspect below or the method described in Synthetic Example 4 below. Compound 1 may be obtained in any way, but is preferably produced in accordance with, for example, the method of the second aspect below.

The sodium base is used in order to make compound 1 into the sodium salt (compound 3). The sodium base is, for example, at least one selected from the group consisting of sodium C1-6 alkoxides, sodium hydroxide, sodium hydride, and sodium hexamethyldisilazide, or comprises the at least one. Preferably, the sodium base is at least one selected from the group consisting of sodium tert-butoxide, sodium tert-pentoxide, sodium hydroxide, sodium hydride, sodium methoxide, sodium ethoxide, and sodium hexamethyldisilazide, or comprises the at least one. More preferably, the sodium base is at least one selected from the group consisting of sodium tert-butoxide, sodium tert-pentoxide, and sodium hydroxide, or comprises the at least one. Most preferably, the sodium base is sodium tert-butoxide.

In general formula (A), $X_1$ is preferably a hydroxy group, $R_1$ is preferably a hydrogen atom or a C1-6 alkyl group, $R_2$ is preferably a hydrogen atom or a C1-6 alkyl group, $R_3$ is preferably a hydrogen atom or a C1-6 alkyl group, $R_4$ is preferably a hydrogen atom or a C1-6 alkyl group, and $R_5$ is preferably a hydrogen atom or a C1-6 alkyl group.

The organic acid is preferably at least one selected from the group consisting of o-cresol, m-cresol, p-cresol, phenol, and dibutylhydroxytoluene, more preferably at least one selected from the group consisting of m-cresol, phenol, and dibutylhydroxytoluene, and most preferably m-cresol.

The organic acid is preferably an acid weaker than compound 1. Also, the organic acid preferably has a pKa of 7.0 to 12.0, more preferably 7.0 to 11.0, and even more preferably 8.0 to 11.0, in water at 25°C.

A solubility at 25°C of a sodium salt of the organic acid in a mixed solvent of tetrahydrofuran, acetone, tert-butyl methyl ether, and water (the volume ratio of tetrahydrofuran, acetone, and tert-butyl methyl ether is 8:10:5, and the content of water is 3% by mass with respect to the mass of the mixed solvent) is 3 mmol/L or more, more preferably 0.05 mol/L or more, and even more preferably 0.08 mol/L or more.

A solubility at 25°C of an organic acid in a mixed solvent of tetrahydrofuran, acetone, tert-butyl methyl ether, and water (the volume ratio of tetrahydrofuran, acetone, and tert-butyl methyl ether is 8:10:5, and the content of water is 3% by mass with respect to the mass of the mixed solvent) is 0.05 mol/L or more, more preferably 0.08 mol/L or more, and even more preferably 0.10 mol/L or more.

The molar equivalent of the organic acid mixed in step (A-1) is preferably 0.1 to 3, more preferably 0.1 to 1.5, and even more preferably 0.3 to 0.6, with respect to the sodium base.

The molar equivalent of the sodium base mixed in step (A-1) is preferably 0.3 to 3, more preferably 0.5 to 1.5, and even more preferably 0.8 to 1.2, with respect to compound 1.

The first solvent is, for example, at least one selected from the group consisting of ether solvents, ketone solvents, and amide solvents, or comprises the at least one. Examples of the ether solvents include diethyl ether,

tetrahydrofuran, 2-methyltetrahydrofuran, 1,4-dioxane, 1,3-dioxolane, 1,2-dimethoxyethane, diisopropyl ether, cyclopentyl methyl ether, tert-butyl methyl ether, 4- methyltetrahydropyran, diglyme, triglyme, and tetraglyme. Examples of the ketone solvents include methyl ethyl ketone, acetone, methyl isobutyl ketone, and cyclohexanone. Examples of the amide solvents include dimethylformamide, dimethylacetamide, and N-methyl-2-pyrrolidone.

The first solvent is preferably at least one selected from the group consisting of tetrahydrofuran, acetone, and 1,3-dimethyl-2-imidazolidinone, or comprises the at least one. The first solvent is more preferably at least one selected from the group consisting of tetrahydrofuran and acetone, or comprises the at least one.

The temperature of the first solvent in step (A-1) is, for example, -25°C to 40°C, preferably -15°C to 15°C, more preferably -10°C to 10°C, and even more preferably -5°C to 5°C.

The volume of the first solvent in step (A-1) is preferably 6 to 16 mL, more preferably 8 to 14 mL, and even more preferably 10 to 12 mL, per 1 g of the mass of compound 3 produced in step (A-1).

Step (A-1) can be carried out in a vessel, for example. For example, in the case where step (A-1) is carried out in a vessel, the above-described (a), (b) and (c) and the first solvent may be added to the vessel in any order, and for example, may be added at the same time. Alternatively, for example, the above-described (b) and (c) may be mixed first, and then this may be mixed with the above-described (a) and the first solvent. Also, for example, each of the above-described (a), (b), and (c) may take the form of a solution at the time of addition.

In some preferred embodiments, the method of the first aspect of the present disclosure further comprises both or either of steps (A-2) and (A-3) below:

(A-2) mixing the mixture obtained in step (A-1) with a second solvent; and
(A-3) obtaining compound 3 from the mixture obtained in step (A-1) or (A-2).

The second solvent is, for example, at least one selected from the group consisting of ether solvents, ketone solvents, and hydrocarbon solvents, or comprises the at least one. Examples of the ether solvents include tert-butyl methyl ether, cyclopentyl methyl ether, 4-methyltetrahydropyran, tetrahydrofuran, diethyl ether, 2-methyltetrahydrofuran, 1,4-dioxane, 1,3-dioxolane, 1,2-dimethoxyethane, diisopropyl ether, diglyme, triglyme, and tetraglyme. Examples of the ketone solvents include acetone, methyl ethyl ketone, methyl isobutyl ketone, and cyclohexanone. Examples of the hydrocarbon solvents include n-hexane, n-heptane, n-octane, n-decane, n-dodecane, 2,3-dimethylhexane, 2-methylheptane, 2-methylhexane, 3-methylhexane, and cyclohexane.

The second solvent is preferably at least one selected from the group consisting of acetone, methyl ethyl ketone, tert-butyl methyl ether, cyclopentyl methyl ether, and n-heptane, or comprises the at least one. The second solvent is more preferably at least one selected from the group consisting of acetone and tert-butyl methyl ether, or comprises the at least one.

The temperature of the second solvent in step (A-2) is, for example, -25°C to 50°C, preferably 0°C to 45°C, more preferably 5°C to 40°C, and even more preferably 25°C to 40°C.

The volume of the second solvent in step (A-2) is preferably 0.3 to 3 times, more preferably 0.5 to 2 times, and even more preferably 0.7 to 1.3 times the volume of the first solvent.

In step (A-3), compound 3 is obtained from the mixture obtained in step (A-2) by solid-liquid separation, for example. The solid-liquid separation can be carried out by, for example, filtration or centrifugation.

A second aspect of the present disclosure provides a method for producing 2-(4-cyclopropyl-2-fluoroanilino)-3,4-difluoro-5-[[3-fluoro-2-(methylsulfamoylamino)pyridin-4-yl]methyl]benzamide (compound 1), the method comprising steps (B-1), (B-2), and (B-3) below:

(B-1) mixing (d), (e), and (f) below to obtain a mixture containing compound 1:

(d) 5-[(2-amino-3-fluoropyridin-4-yl)methyl]-2-(4-cyclopropyl-2-fluoroanilino)-3,4-difluorobenzamide (compound 2);
(e) N-methylsulfamoyl chloride; and
(f) an aqueous solution of an inorganic salt;

(B-2) removing an aqueous layer from the mixture obtained in step (B-1) by a separating operation; and
(B-3) mixing the mixture obtained in step (B-2) with a fourth solvent to remove said inorganic salt.

Compound 2 (in the present disclosure, also referred to as "compound 5A") is represented by the formula below.

[Chemical Formula 5]

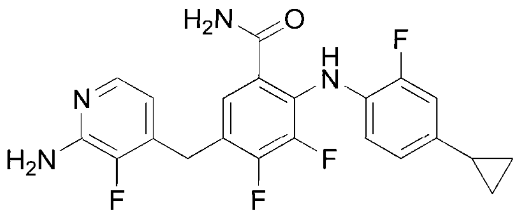

2

Compound 2 can be produced in accordance with, for example, the method described in Synthetic Example 3 (3-1) or 4 (4-2) below. Compound 2 may be obtained in any way.

N-Methylsulfamoyl chloride, for example, is a commercially available reagent.

The aqueous solution of an inorganic salt is preferably at least one selected from the group consisting of an aqueous sodium chloride solution, an aqueous ammonium chloride solution, an aqueous sodium dihydrogen phosphate solution, and an aqueous potassium dihydrogen phosphate solution, more preferably at least one selected from the group consisting of an aqueous sodium chloride solution and an aqueous ammonium chloride solution, and most preferably an aqueous sodium chloride solution.

The concentration of the inorganic salt in the aqueous solution is, for example, 5 to 25% by mass, and preferably 8 to 23% by mass.

Step (B-1) is preferably carried out in a third solvent.

The third solvent is, for example, at least one selected from the group consisting of ether solvents, ketone solvents, and ester solvents, or comprises the at least one. Examples of the ether solvents include tert-butyl methyl ether, cyclopentyl methyl ether, 4-methyltetrahydropyran, tetrahydrofuran, diethyl ether, 2-methyltetrahydrofuran, 1,4-dioxane, 1,3-dioxolane, 1,2-dimethoxyethane, diisopropyl ether, diglyme, triglyme, and tetraglyme. Examples of the ketone solvents include acetone, methyl ethyl ketone, methyl isobutyl ketone, and cyclohexanone. Examples of the ester solvents include ethyl acetate, butyl acetate, isopropyl acetate, ethylene glycol diacetate, and propylene glycol monomethyl ether acetate.

The third solvent is preferably at least one selected from the group consisting of tetrahydrofuran, 2-methyltetrahydrofuran, 4-methyltetrahydropyran, acetone, methyl ethyl ketone, ethyl acetate, and isopropyl acetate, or comprises the at least one. The third solvent is most preferably tetrahydrofuran.

The temperature of the third solvent in step (B-1) is, for example, -20°C to 10°C, preferably -20°C to 8°C, more preferably -20°C to 5°C, and even more preferably -20°C to 0°C.

Step (B-1) can be carried out in a vessel, for example. For example, in the case where step (B-1) is carried out in the third solvent in a vessel, the above-described (d), (e) and (f) and the third solvent may be added to the vessel in any order, and for example, may be added at the same time. Alternatively, for example, the above-described (e) and (f) may be mixed first, and then this may be mixed with the above-described (d) and the third solvent. Also, for example, each of the above-described (d) and (e) may take the form of a solution at the time of addition.

The fourth solvent is, for example, at least one selected from the group consisting of ketone solvents, ether solvents, ester solvents, hydrocarbon solvents, aromatic solvents, alcohol solvents, and halogen solvents, or comprises the at least one. The fourth solvent is preferably at least one selected from the group consisting of ketone solvents and ether solvents, or comprises the at least one. Examples of the ketone solvents include acetone, methyl ethyl ketone, methyl isobutyl ketone, and cyclohexanone. Examples of the ether solvents include tert-butyl methyl ether, cyclopentyl methyl ether, 4-methyltetrahydropyran, tetrahydrofuran, diethyl ether, 2-methyltetrahydrofuran, 1,4-dioxane, 1,3-dioxolane, 1,2-dimethoxyethane, diisopropyl ether, diglyme, triglyme, and tetraglyme. Examples of the ester solvents include ethyl acetate, butyl acetate, isopropyl acetate, ethylene glycol diacetate, and propylene glycol monomethyl ether acetate. Examples of the hydrocarbon solvents include n-hexane, n-heptane, n-octane, n-decane, n-dodecane, 2,3-dimethylhexane, 2-methylheptane, 2-methylhexane, 3-methylhexane, and cyclohexane. Examples of the aromatic solvents include toluene and xylene. Examples of the alcohol solvents include methanol, ethanol, propanol, isopropanol, and butanol. Examples of the halogen solvents include dichloromethane and chloroform.

The fourth solvent is more preferably a solvent that does not dissolve sodium chloride and dissolves compound 1, and is even more preferably at least one selected from the group consisting of acetone, methyl ethyl ketone, and tetrahydrofuran, or comprises the at least one.

The temperature of the fourth solvent in step (B-3) is, for example, -20°C to 25°C, preferably -15°C to 20°C, more preferably -10°C to 15°C, and even more preferably -5°C to 10°C.

Step (B-3) can be carried out in a vessel, for example. For example, in the case where step (B-3) is carried out in a vessel, the mixture obtained in step (B-2) and the fourth solvent may be added to the vessel in any order, and for example,

may be added at the same time.

In step (B-3), the removal of the inorganic salt can be carried out by, for example, using a separating operation.

The method of the second aspect may be performed as one step in the method for producing compound 3.

That is, a third aspect of the present disclosure provides a method for producing a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), the method comprising step (C-1) below:

(C-1) producing 2-(4-cyclopropyl-2-fluoroanilino)-3,4-difluoro-5-[[3-fluoro-2-(methylsulfamoylamino)pyridin-4-yl]methyl]benzamide (compound 1) by the method of the second aspect.

In one preferred embodiment, the method of the third aspect further comprises producing compound 3 from compound 1 obtained in step (C-1), using a sodium base. This step can be carried out in accordance with, for example, the method described in Synthetic Example 4 below.

The sodium base is used in order to make compound 1 into the sodium salt (compound 3). The sodium base is, for example, at least one selected from the group consisting of sodium C1-6 alkoxides, sodium hydroxide, sodium hydride, and sodium hexamethyldisilazide, or comprises the at least one. Preferably, the sodium base is at least one selected from the group consisting of sodium tert-butoxide, sodium tert-pentoxide, sodium hydroxide, sodium hydride, sodium methoxide, sodium ethoxide, and sodium hexamethyldisilazide, or comprises the at least one. More preferably, the sodium base is at least one selected from the group consisting of sodium tert-butoxide, sodium tert-pentoxide, and sodium hydroxide, or comprises the at least one. Most preferably, the sodium base is sodium tert-butoxide.

The molar equivalent of the sodium base used in the above step is preferably 0.3 to 3, more preferably 0.5 to 1.5, and even more preferably 0.8 to 1.2, with respect to compound 1.

Also, in another preferred embodiment, the method of the third aspect further comprises step (C-2) below:

(C-2) producing compound 3 by the method of the first aspect, using compound 1 obtained in step (C-1).

The method of the first or third aspect makes it possible to obtain a product with a high content of compound 3 (for example, 97.0% by mass or more, 98.0% by mass or more, or 99.0% by mass or more).

That is, a fourth aspect of the present disclosure provides a composition comprising a sodium salt of 2-(4-cyclopropyl-2-fluoroanilino)-3,4-difluoro-5-[[3-fluoro-2-(methylsulfamoylamino)pyridin-4-yl]methyl]benzamide (compound 3), wherein the content of compound 3 is 98.0% by mass or more with respect to the mass of said composition.

In one preferred embodiment, the composition of the fourth aspect is a composition in which the content of compound 3 is 99.0% by mass or more with respect to the mass of said composition.

Also, in another preferred embodiment, the composition of the fourth aspect is a composition in which the peak area of compound 3 obtained by high-performance liquid chromatography (HPLC) analysis at 278 nm for said composition is 99.0% or more with respect to the sum of peak areas of all substances detected in said HPLC analysis.

In the case where compound 3 is produced by the method of the first or third aspect, 2-(4-cyclopropyl-2-fluoroanilino)-3,4-difluoro-5-[[3-fluoro-2-(methylsulfamoylamino)pyridin-4-yl]methyl]benzoic acid (compound 4) may be produced, but the amount of compound 4 produced is small and the peak area of compound 4 obtained by high-performance liquid chromatography (HPLC) analysis at 278 nm is 0.20% or less with respect to the peak area of compound 3 obtained by the HPLC analysis.

That is, a fifth aspect of the present disclosure provides a composition comprising a sodium salt of 2-(4-cyclopropyl-2-fluoroanilino)-3,4-difluoro-5-[[3-fluoro-2-(methylsulfamoylamino)pyridin-4-yl]methyl]benzamide (compound 3) and 2-(4-cyclopropyl-2-fluoroanilino)-3,4-difluoro-5-[[3-fluoro-2-(methylsulfamoylamino)pyridin-4-yl]methyl]benzoic acid (compound 4), wherein the peak area of compound 4 obtained by high-performance liquid chromatography (HPLC) analysis at 278 nm for said composition is 0.20% or less with respect to the peak area of compound 3 obtained by said HPLC analysis.

In one preferred embodiment, the composition of the fifth aspect is a composition in which the peak area of compound 4 obtained by high-performance liquid chromatography (HPLC) analysis at 278 nm for said composition is 0.05% or less with respect to the peak area of compound 3 obtained by said HPLC analysis.

Compound 4 is represented by the formula below.

[Chemical Formula 6]

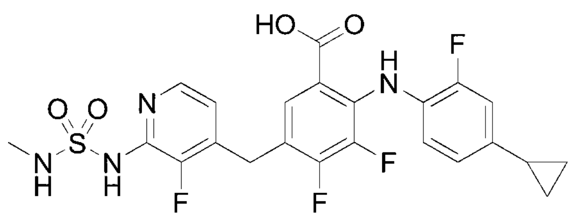

4

Compound 3 is useful in the treatment or prevention of cancer. That is, in one embodiment, the composition of the fifth aspect is a pharmaceutical composition. Also, in one embodiment, the pharmaceutical composition is a pharmaceutical composition for the treatment or prevention of cancer.
The method of the first or third aspect is also a method for producing the composition of the fourth or fifth aspect.
The method of the first or third aspect is also a method for obtaining a product with a high content of compound 3.
The content of compound 3 in the product can be determined by, for example, the quantitative NMR method using a commercially available internal standard substance (for example, dimethyl sulfone). The quantitative NMR measurement can be carried out under the conditions below, for example.
Deuterated solvent; dimethyl sulfoxide-$d_6$ and deuterated water ($D_2O$) were used in a volume ratio of 9:1.

Observed nucleus: $^1H$
Pulse angle: 90°
Delay time: 60 sec
Digital resolution: 0.25 Hz
Spinning: off
Number of scans: 32

The content of compound 3 in the product can also be determined by HPLC analysis in the case where the content of compound 3 in the specimen is known. The HPLC analysis can be carried out using the analysis conditions 1 listed in Table 1 below, for example. The content of compound 3 in the product can be calculated according to the expression below:

the content of compound 3 in the product (% by mass) = [the mass of the specimen subjected to the HPLC analysis (mg) / the mass of the product subjected to the HPLC analysis (mg)] × [the peak area of compound 3 obtained by the HPLC analysis for the product / the peak area of compound 3 obtained by the HPLC analysis for the specimen] × the content of compound 3 in the specimen (% by mass).

Seed crystals of a compound or a salt thereof may be used in connection with carrying out inventions of the present disclosure. Generally, seed crystals can be obtained by a method well known to those skilled in the art, such as by: cooling a solution of the compound or salt; adding a solvent with low solubility for the compound or salt (i.e., a poor solvent); rubbing with a spatula the wall of a vessel containing a solution of the compound or salt; or concentrating a solution of the compound or salt under reduced pressure after purification by silica gel column chromatography.
The following are examples of abbreviations used throughout the present specification and their meanings.

AA: Ammonium acetate
BHT: Dibutylhydroxytoluene
DCM: Dichloromethane
DIPEA: N,N-Diisopropylethylamine
DMA: N,N-Dimethylacetamide
DMAP: N,N-Dimethylaminopyridine
DMF: N,N-Dimethylformamide
DMSO: Dimethyl sulfoxide
EDC·HCl: 1-(3-Dimethylaminopropyl)-3-ethylcarbodiimide hydrochloride
EtOH: Ethanol
FA: Formic acid
HOOBt: 3,4-Dihydro-3-hydroxy-4-oxo-1,2,3-benzotriazine
2-MeTHF: 2-Methyltetrahydrofuran
MeCN: Acetonitrile
MeOH: Methanol
NMP: N-Methyl-2-pyrrolidone
RuPhos-Pd-G3: (2-Dicyclohexylphosphino-2',6'-diisopropoxy-1,1'-biphenyl)[2-(2'-amino-1,1'-biphenyl)]palladium(II) methanesulfonate
TBME: *tert*-Butyl methyl ether
TFA: Trifluoroacetic acid
THF: Tetrahydrofuran
Xantphos: 4,5-Bis(diphenylphosphino)-9,9-dimethylxanthene
XPhos-Pd-G3: (2-Dicyclohexylphosphino-2',4',6'-triisopropyl-1,1'-biphenyl)[2-(2'-amino-1,1'-biphenyl)]palladium(II) methanesulfonate

As used in the present disclosure, "room temperature" means a temperature of about 20°C to about 25°C.

Examples

The present disclosure will now be explained in greater detail based on examples, with the understanding that the present disclosure is in no way limited to the examples.
Unless otherwise noted, starting materials and reagents used in the Examples below were obtained commercially or produced by known methods.
In the Examples below, high-performance liquid chromatography (HPLC) analysis was carried out using one of the analysis conditions listed in Table 1 below.

[Table 1-1]

Table 1

| Analysis conditions | Apparatus | Column | Column temperature | Detection wavelength (PDA) |
|---|---|---|---|---|
| 1 | Waters H-Class | ZORBAX ECLIPSE Plus C18 2.1 mm I.D. × 50 mm L, 1.8 μm | 35°C | 278 nm |
| 2 | Waters H-Class | ZORBAX ECLIPSE Plus C18 2.1 mm I.D. × 50 mm L, 1.8 μm | 35°C | 278 nm |

[Table 1-2]

Table 1 (cont.)

| Analysis conditions | Mobile phase | Gradient | | Flow rate (mL/min) |
|---|---|---|---|---|
| | | Time after injection (min) | A/B | |
| 1 | A) 0.05% TFA/ $H_2O$<br>B) 0.05% TFA/ MeCN | 0-5.0<br>5.0-8.0<br>8.0-9.0 | 65/35 → 50/50<br>50/50 → 0/100<br>0/100 | 0.8 |
| 2 | A) 0.01% TFA/ $H_2O$<br>B) 0.01% TFA/ MeCN | 0-5.0<br>5.0-8.0<br>8.0-9.0 | 67/33 → 50/50<br>50/50 → 0/100<br>0/100 | 0.8 |

The content of water in the solvent was measured by the Karl Fischer method (electrolytic method) using a moisture meter CA-200 manufactured by Mitsubishi Chemical Analytech Co., Ltd.
The NMR measurement was performed using the nuclear magnetic resonance spectrometer JNM-ECZ-500R manufactured by JEOL Ltd.

(Example 1)

Synthesis of 5-bromo-2-(4-cyclopropyl-2-fluoroanilino)-3,4-difluorobenzamide

[Chemical Formula 7]

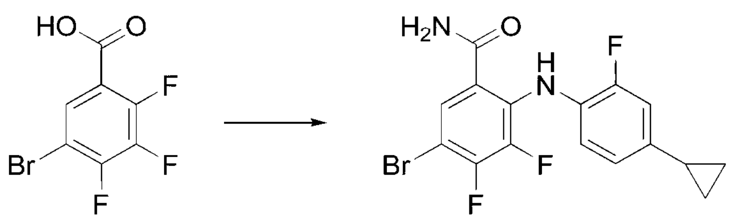

A reaction vessel in which a 24% lithium bis(trimethylsilyl)amide THF solution (94.7 kg, 136 mol) had been placed

was cooled to an external temperature of -15°C, and a solution of 4-cyclopropyl-2-fluoroaniline (8.1 kg, 53.5 mol) in THF (18.7 kg) was added dropwise at an internal temperature of 5°C or lower. After the reaction vessel was thoroughly washed with THF (4.7 kg), a solution of 5-bromo-2,3,4-trifluorobenzoic acid (10.5 kg, 41.2 mol) in THF (65.3 kg) was added dropwise while keeping the internal temperature at 5°C or lower, and the mixture was stirred for 30 minutes. Then 5 M hydrochloric acid (88.7 kg) was added to the reaction mixture, the temperature was increased to room temperature, and extraction was performed with isopropyl acetate (45.9 kg). The organic layer was washed sequentially once with 2 M hydrochloric acid (42.8 kg) and twice with a 15% aqueous sodium chloride solution (52.5 kg), and concentrated under reduced pressure. THF (74.8 kg) was added to the resulting concentrated solution, which was then concentrated until the total volume reached 75 L. Again, THF (74.8 kg) was added to the concentrated solution, which was then concentrated until the total volume reached 75 L. Then, acetonitrile (100 kg) and THF (31.6 kg) were added sequentially to the concentrated solution. To the resulting solution, 1,1'-carbonyldiimidazole (10.0 kg, 48.8 mmol) was added, and the mixture was stirred at room temperature for 1 hour. To the reaction mixture, 28% ammonia water (14.5 kg) was added, the mixture was stirred at room temperature for 1.5 hours, and the resulting suspension was heated to 60°C. After confirming the dissolution of the suspended material, the solution was cooled to 25°C, and seed crystals of 5-bromo-2-(4-cyclopropyl-2-fluoroanilino)-3,4-difluorobenzamide (80 g) and water (191 kg) were added successively. The precipitated crystals were filtered off, and the filtration residue was washed twice with a liquid mixture of acetonitrile (18.7 kg) and water (23.9 kg) and then dried at an external temperature of 40°C under reduced pressure to give the title compound (13.84 kg, 87.3% yield).

The above seed crystals were produced in accordance with the method described in Synthetic Example 2 (2-2) below.

(Example 2)

<u>Production of a sodium salt of 2-(4-cyclopropyl-2-fluoroanilino)-3,4-difluoro-5-[[3-fluoro-2-(methylsulfamoylamino)pyridin-4-yl]methyl]benzamide (compound 3)</u>

[Chemical Formula 8]

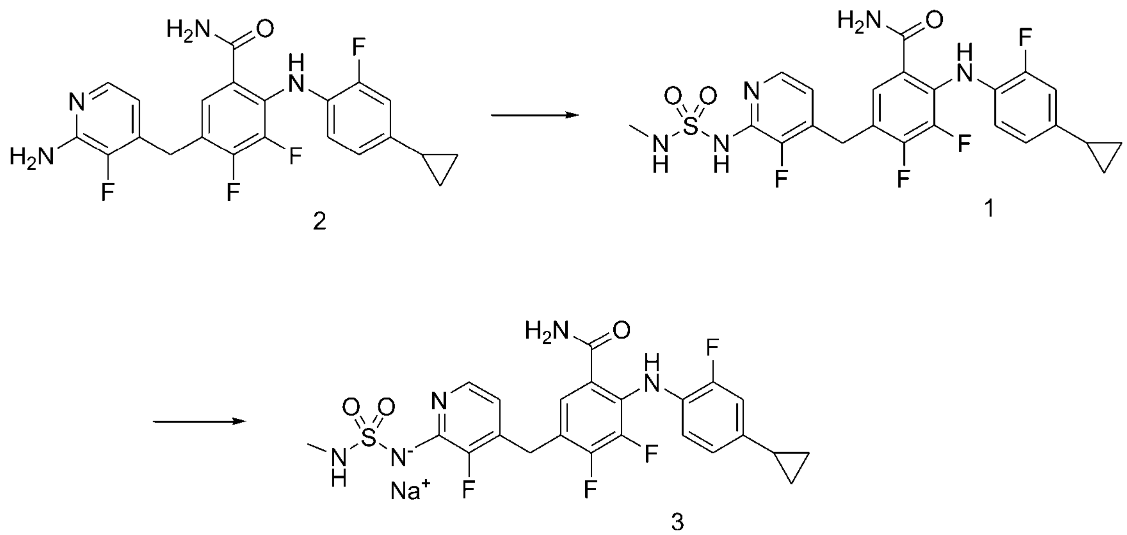

2

1

3

(2-1) Effects of various additives

To a reaction vessel, 5-[(2-amino-3-fluoropyridin-4-yl)methyl]-2-(4-cyclopropyl-2-fluoroanilino)-3,4-difluorobenzamide (compound 2) (1.00 g, 2.32 mmoL) and THF (10.0 mL) were added, and the reaction vessel was deaerated under reduced pressure while stirring and purged with nitrogen. Pyridine (0.564 mL, 6.97 mmol) was added to the resulting solution, which was cooled to a temperature of -20°C to 0°C, and N-methylsulfamoyl chloride was then added stepwise while checking the conversion by HPLC (analysis conditions 1). After confirming that the conversion reached 95% or more, a 10% aqueous sodium chloride solution (5.00 g) was added while keeping the temperature of the reaction solution at 10°C or lower. The organic layer obtained by separation was washed sequentially with a 10% aqueous sodium chloride solution (5.0 g) and a 20% aqueous sodium chloride solution (5.00 g). While keeping the resulting solution at 10°C or lower, acetone (3.7 mL) was added, and THF was added to the liquid mixture to adjust the volume ratio of acetone and THF in the solution

to 3:8. Then, the resulting suspension was filtered to obtain a clear solution. After adding water to adjust the content of water in the solution to about 5% by mass, 2 M sodium tert-butoxide (1.11 mL, 2.21 mmol) was added dropwise with or without the addition of an additive (acetaminophen, BHT, phenol, or m-cresol), while maintaining the temperature of the reaction solution in the range of 20°C to 40°C. Then, a suspension of seed crystals of compound 3 (12 mg) in acetone (0.24 mL) was added at 40°C. Acetone (2.2 mL) was added over 30 minutes and the mixture was stirred for 1 hour. Furthermore, acetone (6.1 mL) was added over 20 minutes and the mixture was stirred for 30 minutes. Then, TBME (6.1 mL) was added over 20 minutes and the mixture was stirred for another 30 minutes. The resulting suspension was cooled to 25°C over 30 minutes and stirred for another 30 minutes. The precipitated solid was filtered off, and the filtration residue was washed twice with a mixed solvent of acetone (3.7 mL) and TBME (3.7 mL), and then dried under reduced pressure to give compound 3. For the resulting sample of compound 3 (hereinafter, referred to as "sample 3a"), HPLC analysis was carried out using the analysis conditions 1.

Compound 2 above was produced from 5-bromo-2-(4-cyclopropyl-2-fluoroanilino)-3,4-difluorobenzamide obtained in Example 1 above in accordance with the method described in Synthetic Example 3 (3-1) below.

The above seed crystals were produced from 5-bromo-2-(4-cyclopropyl-2-fluoroanilino)-3,4-difluorobenzamide obtained in Example 1 above in accordance with the method described in Synthetic Examples 3 (3-1) and 4 (4-1) below.

The conversion was calculated according to the expression below:

the conversion (%) = [(the peak area of compound 1 + the peak area of compound X + the peak area of compound Y)/(the peak area of compound 1 + the peak area of compound 2 + the peak area of compound X + the peak area of compound Y)] $\times$ 100 (%).

The HPLC retention time was about 3.26 min for compound 1, 1.02 min for compound 2, 3.54 min for compound X, and 4.05 min for compound Y.

The structural formulas of compound X and compound Y are as follows.

[Chemical Formula 9]

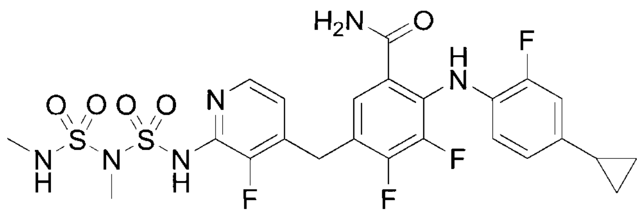

X

[Chemical Formula 10]

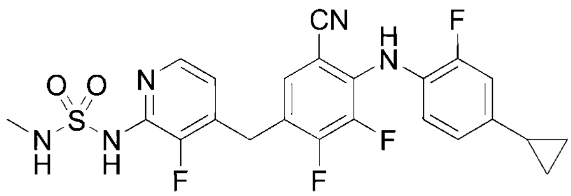

Y

The results of the HPLC analysis for sample 3a are shown in Table 2. Table 2 shows the content of compound 3 in sample 3a, the HPLC purity of compound 3 in sample 3a (the percentage of the peak area of compound 3 with respect to the sum of the peak areas of all substances detected in the HPLC analysis for sample 3a), and the amount of impurity, 2-(4-cyclopropyl-2-fluoroanilino)-3,4-difluoro-5-[[3-fluoro-2-(methylsulfamoylamino)pyridin-4-yl]methyl]benzoic acid (compound 4), in sample 3a (relative amount to compound 3).

The content of compound 3 in sample 3a was calculated from the content of compound 3 in the specimen of compound 3 (hereinafter, referred to as "specimen 3") according to the expression below:

the content of compound 3 in sample 3a (% by mass) = [the mass of specimen 3 subjected to the HPLC analysis (mg) / the mass of sample 3a subjected to the HPLC analysis (mg)] × [the peak area of compound 3 obtained by the HPLC analysis for sample 3a / the peak area of compound 3 obtained by the HPLC analysis for specimen 3] × the content of compound 3 in specimen 3 (% by mass).

Specimen 3 was obtained by crushing with a jet mill the sample produced from 5-bromo-2-(4-cyclopropyl-2-fluoroanilino)-3,4-difluorobenzamide obtained in Example 1 above in accordance with the method described in Synthetic Examples 3 (3-1) and 4 (4-1) below. The HPLC analysis for specimen 3 was carried out using the analysis conditions 1.
The content of compound 3 in specimen 3 was determined by the quantitative NMR method using dimethyl sulfone as the internal standard substance. The quantitative NMR measurement was carried out under the conditions below.
Deuterated solvent; dimethyl sulfoxide-$d_6$ and deuterated water ($D_2O$) were used in a volume ratio of 9:1.

Observed nucleus: $^1H$
Pulse angle: 90°
Delay time: 60 sec
Digital resolution: 0.25 Hz
Spinning: off
Number of scans: 32

The HPLC purity of compound 3 in sample 3a was calculated according to the expression below:

the HPLC purity of compound 3 in sample 3a (%) = 100 (%) - [the sum of the peak areas of all impurities detected in the HPLC analysis for sample 3a / the sum of the peak areas of all substances detected in the HPLC analysis for sample 3a] × 100 (%).

The amount of compound 4 in sample 3a (relative amount to compound 3) was calculated according to the expression below:

the amount of compound 4 in sample 3a (%) = [the peak area of compound 4 obtained by the HPLC analysis for sample 3a / the peak area of compound 3 obtained by the HPLC analysis for sample 3a] × 100 (%).

The structural formula of compound 4 is as follows.

[Chemical Formula 11]

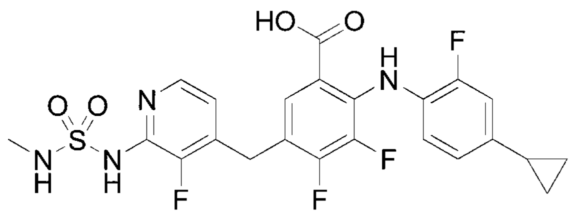

4

Table 2 also shows the pKa of the additive, the solubility of the sodium salt of the additive, and the solubility of the additive.
The pKa values shown in Table 2 are the pKa values in water described in the literature below. The pKa values of acetaminophen, phenol, and m-cresol are the pKa values in water at 25°C.

Acetaminophen: Org. Biomol. Chem., 2009, 7, 5103.
BHT: IUPAC Chemical Data Series, No. 23; Pergamon Press: Oxford, UK, 1979.
Phenol: Handbook of Biochemistry and Selected Data for Molecular Biology, 2nd ed.; CRC: Cleveland, OH, 1970.
m-Cresol: Handbook of Chemistry and Physics, 85th ed.; CRC: Boca Raton, FL, 2004-2005.

The solubility of the additive or its sodium salt was measured as follows. A mixed solvent of tetrahydrofuran, acetone, tert-butyl methyl ether, and water (the volume ratio of tetrahydrofuran, acetone, and tert-butyl methyl ether is

8:10:5, and the content of water is 3% by mass with respect to the mass of the mixed solvent) was prepared. The temperature of the solvent was brought to 25°C, the additive (acetaminophen, BHT, phenol, or m-cresol) or its sodium salt was added, and the resulting suspension was stirred for 6 hours or longer. The supernatant was subjected to HPLC analysis using the analysis conditions 1.

[Table 2-1]

| Table 2 | | | | |
|---|---|---|---|---|
| Additive | Amount added | Content of compound 3 in sample 3a | HPLC purity of compound 3 in sample 3a | Amount of compound 4 in sample 3a (Relative amount to compound 3) |
| Absent | - | 97.9% by mass | 99.6% | 0.22% |
| Acetaminophen | 0.106 g (0.698 mmol) | 92.6% by mass | Unmeasured | Unmeasured |
| BHT | 0.154 g (0.698 mmol) | 99.3% by mass | 99.4% | 0.25% |
| Phenol | 0.066 g (0.70 mmol) | 99.5% by mass | 99.7% | Undetected |
| m-Cresol | 0.075 g (0.70 mmol) | 99.6% by mass | 99.6% | 0.02% |

[Table 2-2]

| Table 2 (cont.) | | | |
|---|---|---|---|
| Additive | pKa | Solubility of sodium salt of additive | Solubility of additive |
| Absent | - | - | - |
| Acetaminophen | 9.78 | 0.04 mol/L | 0.07 mol/L or more |
| BHT | 12.2 | 0.07 mol/L or more | 0.05 mol/L or more |
| Phenol | 9.95 | 3 mmol/L | 0.08 mol/L or more |
| m-Cresol | 10.09 | 0.05 mol/L or more | 0.05 mol/L or more |

As the above results show, the addition of BHT, phenol, or m-cresol provides a product with a higher content of compound 3 compared to the case where no additive was added and the case where acetaminophen was added.

Note that, in the case where acetaminophen was added, a residual contaminant was observed in sample 3a. Also, in the case where phenol was added, the peak of compound 4 was not detected.

(2-2) Investigation of method for removing inorganic salt

(1) Removal of inorganic salt by concentration

To a reaction vessel, 5-[(2-amino-3-fluoropyridin-4-yl)methyl]-2-(4-cyclopropyl-2-fluoroanilino)-3,4-difluorobenzamide (compound 2) (1.00 g, 2.32 mmoL) and THF (10.0 mL) were added, and the reaction vessel was deaerated under reduced pressure while stirring and purged with nitrogen. Pyridine (0.564 mL, 6.97 mmol) was added to the resulting solution, which was cooled to a temperature of -20°C to 0°C, and N-methylsulfamoyl chloride was then added stepwise while checking the conversion by HPLC (analysis conditions 2). After confirming that the conversion reached 95% or more, a 10% aqueous sodium chloride solution (5.00 g) was added while keeping the temperature of the reaction solution at 10°C or lower. The organic layer obtained by separation was washed sequentially with a 10% aqueous sodium chloride solution (5.0 g) and a 20% aqueous sodium chloride solution (5.00 g). An operation in which THF (15.0 mL) was added and the mixture was concentrated to a total volume of 5 mL was carried out twice at 40°C, and the resulting suspension was filtered to make a clear solution. Acetone (3.7 mL) and THF were added to adjust the volume ratio of acetone and THF in the solution to 3:8. After adding water to adjust the content of water in the solution to about 5% by mass, 2 M sodium tert-butoxide (1.10 mL, 2.21 mmol) was added dropwise with the addition of m-cresol, while maintaining the temperature of the reaction solution in the range of 20°C to 40°C. Then, a suspension of seed crystals of compound 3 (12 mg) in acetone (0.20 mL) was added at 40°C. Acetone (2.5 mL) was added over 30 minutes and the mixture was stirred for 1 hour. Furthermore, acetone (6.0 mL) was added over 20 minutes and the mixture was stirred for 30 minutes. Then, TBME (6.0 mL) was added over 20 minutes and the mixture was stirred for another 30 minutes. The resulting suspension was cooled to 25°C over 30 minutes and stirred for another 30 minutes. The precipitated solid was filtered off, and the filtration residue was washed

twice with a mixed solvent of acetone (3.5 mL) and TBME (3.5 mL), and then dried under reduced pressure to give compound 3. For the resulting sample of compound 3 (hereinafter, referred to as "sample 3c"), HPLC analysis was carried out using the analysis conditions 2.

Compound 2 above and the seed crystals above were produced in the same manner as in (2-1) above. The conversion was also calculated in the same manner as in (2-1) above.

The content of compound 3 in sample 3c was 99.5% by mass. The content of compound 3 in sample 3c was calculated in the same manner as the content of compound 3 in sample 3a above.

(2) Removal of inorganic salt by solvent addition

To a reaction vessel, 5-[(2-amino-3-fluoropyridin-4-yl)methyl]-2-(4-cyclopropyl-2-fluoroanilino)-3,4-difluorobenzamide (compound 2) (1.00 g, 2.32 mmoL) and THF (10.0 mL) were added, and the reaction vessel was deaerated under reduced pressure while stirring and purged with nitrogen. Pyridine (0.564 mL, 6.97 mmol) was added to the resulting solution, which was cooled to a temperature of -20°C to 0°C, and N-methylsulfamoyl chloride was then added stepwise while checking the conversion by HPLC (analysis conditions 2). After confirming that the conversion reached 95% or more, a 10% aqueous sodium chloride solution (5.00 g) was added while keeping the temperature of the reaction solution at 10°C or lower. The organic layer obtained by separation was washed sequentially with a 10% aqueous sodium chloride solution (5.0 g) and a 20% aqueous sodium chloride solution (5.00 g). While keeping the resulting solution at 10°C or lower, methyl ethyl ketone (3.7 mL) or 2-MeTHF (3.7 mL) was added, and THF was added to the liquid mixture to adjust the volume ratio of methyl ethyl ketone or 2-MeTHF and THF in the solution to 3:8. Then, the resulting suspension was filtered to obtain a clear solution. After adding water to adjust the content of water in the solution to about 5% by mass, 2 M sodium tert-butoxide (1.10 mL, 2.21 mmol) was added dropwise with the addition of m-cresol, while maintaining the temperature of the reaction solution in the range of 20°C to 40°C. Then, a suspension of seed crystals of compound 3 (12 mg) in acetone (0.20 mL) was added at 40°C. Acetone (2.5 mL) was added over 30 minutes and the mixture was stirred for 1 hour. Furthermore, acetone (6.0 mL) was added over 20 minutes and the mixture was stirred for 30 minutes. Then, TBME (6.0 mL) was added over 20 minutes and the mixture was stirred for another 30 minutes. The resulting suspension was cooled to 25°C over 30 minutes and stirred for another 30 minutes. The precipitated solid was filtered off, and the filtration residue was washed twice with a mixed solvent of acetone (3.5 mL) and TBME (3.5 mL), and then dried under reduced pressure to give compound 3. For the resulting sample of compound 3 (hereinafter, referred to as "sample 3d"), HPLC analysis was carried out using the analysis conditions 2.

Compound 2 above and the seed crystals above were produced in the same manner as in (2-1) above. The conversion was also calculated in the same manner as in (2-1) above.

The content of compound 3 in sample 3d was as follows (shown together with the solvent that was added first in the inorganic salt removal step). The content of compound 3 in sample 3d was calculated in the same manner as the content of compound 3 in sample 3a above.

Methyl ethyl ketone: 99.4% by mass
2-MeTHF: 97.4% by mass

(3) Discussion

It was shown that whether the inorganic salt removal was carried out by concentration or by solvent addition, a product with a high content of compound 3 was obtained. It was also shown that, in the case where the inorganic salt removal is carried out by solvent addition, for example, acetone or methyl ethyl ketone is particularly excellent as the solvent (for acetone, see Table 2 (m-cresol) above).

The inorganic salt removal can be done by any of the above methods, but the method by solvent addition is superior in terms of simplicity.

Since the inorganic salt removal by concentration is carried out at a relatively high temperature (for example, 40°C), decomposition of compound 1 may occur during the concentration process, for example, if the concentration takes a long time. The method by solvent addition allows inorganic salt removal at a low temperature (for example, 10°C or lower) and can be said to be advantageous in this respect as well.

(2-3) Production of compound 3

Under a nitrogen atmosphere, after adding THF (21.32 kg) to the reaction vessel, 5-[(2-amino-3-fluoropyridin-4-yl)methyl]-2-(4-cyclopropyl-2-fluoroanilino)-3,4-difluorobenzamide (compound 2) (3.00 kg, 6.97 mol) was added. THF (5.34 kg) was added to the reaction vessel while thoroughly washing the weighing vessel, followed by dropwise addition of pyridine (1.65 kg, 20.9 mol). The solution obtained by stirring was cooled to a temperature of -20°C to -10°C, and while

maintaining the temperature of the solution in this range, N-methylsulfamoyl chloride (0.942 kg in total, 7.27 mol) was added stepwise. A 10% aqueous sodium chloride solution (15.0 kg) was added while keeping the temperature of the reaction solution at 10°C or lower. While maintaining the temperature of the organic layer obtained by separation in the range of - 5°C to 5°C, the organic layer was washed sequentially with a 10% aqueous sodium chloride solution (15.0 kg), a 20% aqueous sodium chloride solution (15.1 kg), and a 20% aqueous sodium chloride solution (15.0 kg). While maintaining the temperature of the organic layer after washing in the range of -5°C to 5°C, acetone (8.63 kg) and THF (8.59 kg) were added sequentially to the organic layer. The resulting suspension was filtered to obtain a clear solution. The internal area of the reaction vessel was washed and filtered with THF (3.24 kg), and the resulting filtrate was combined with the clear solution described above, which was cooled to a temperature of -5°C to 5°C in a crystallization vessel. After adding water to this solution to adjust the content of water in the solution to about 5% by mass, a 2 M solution of sodium tert-butoxide in THF (2.89 kg, 6.66 mol) and m-cresol (0.38 kg, 3.51 mol) (they have been mixed previously under a nitrogen atmosphere) were added dropwise while maintaining the temperature of the solution in the range of -5°C to 5°C. The vessel used for mixing the 2 M solution of sodium tert-butoxide in THF and m-cresol was washed with THF (8.28 kg,) which was added to the crystallization vessel, and the resulting liquid mixture was then heated to a temperature of 25°C to 35°C. Subsequently, seed crystals of compound 3 (18.0 g) were suspended in acetone (0.57 kg) and added to the crystallization vessel, and the resulting suspension was heated to a temperature of 30°C to 40°C and stirred for 30 minutes. The suspension was heated to a temperature of 35°C to 45°C and stirring was continued for 30 minutes. Then, acetone (5.2 kg) was added dropwise to the crystallization vessel over 1 hour or longer, and the mixture was stirred for another 1 hour. Acetone (14.4 kg) was added dropwise over 30 minutes or longer, and the mixture was stirred for another 30 minutes. Then, TBME (13.5 kg) was added over 30 minutes or longer, and the mixture was stirred for another 30 minutes. The resulting suspension was cooled to a temperature of 20°C to 30°C, and the mixture was stirred for another 30 minutes. The precipitated solid was filtered, and the filtration residue was washed twice with a liquid mixture of acetone (8.64 kg) and TBME (8.10 kg), and then dried under reduced pressure to give compound 3 (3.10 kg, 81.1% yield). For the resulting sample of compound 3 (hereinafter, referred to as "sample 3b"), HPLC analysis was carried out using the analysis conditions 2.

Compound 2 above was produced from 5-bromo-2-(4-cyclopropyl-2-fluoroanilino)-3,4-difluorobenzamide obtained in Example 1 above in accordance with the method described in Synthetic Example 3 (3-1) below.

The above seed crystals were obtained by crushing with a jet mill the sample produced from 5-bromo-2-(4-cyclopropyl-2-fluoroanilino)-3,4-difluorobenzamide obtained in Example 1 above in accordance with the method described in Synthetic Examples 3 (3-1) and 4 (4-1) below.

The content and HPLC purity (the percentage of the peak area of compound 3 with respect to the sum of the peak areas of all substances detected in the HPLC analysis for sample 3b) of compound 3 in sample 3b were as follows. The content and HPLC purity of compound 3 in sample 3b was calculated in the same manner as the content and HPLC purity of compound 3 in sample 3a above.

Content: 99.8% by mass
HPLC purity: 99.7%

[Reference Examples]

The following are reference examples (synthetic examples and production examples) pertaining to the production of compounds 1 and 3. In the following reference examples, compound 1 is also referred to as "compound 1A" and compound 2 is also referred to as "compound 5A".

In the reference examples below, "HPLC purity" means the percentage of the peak area of the compound with respect to the sum of the peak areas of all substances detected in the HPLC analysis.

[Synthetic Examples]

In the synthetic examples below, high-performance liquid chromatography (HPLC) analysis was conducted using one of the analysis conditions listed in Table 3 below. Each compound was detected using a photodiode array detector or a mass spectrometer, but another technique such as evaporative light scattering detection may be used.

[Table 3-1]

| Table 3 | | | | |
|---|---|---|---|---|
| Analysis conditions | Apparatus | Column | Column temperature | Detection wavelength (PDA) |
| A | Waters H-Class | InertSustain AQ-C18 HP 2.1 mm I.D. × 50 mm L, 3 μm | 35°C | 210-400 nm |
| B | Acquity SQD/SQD2 | Ascentis Express C18 2.1 mm I.D. × 50 mm L, 2.7 μm | 35°C | 210-400 nm |
| C | Waters H-Class | InertSustain AQ-C18 HP 2.1 mm I.D. × 50 mm L, 3 μm | 35°C | 210-400 nm |
| D | Acquity SQD/SQD2 | Ascentis Express C18 2.1 mm I.D. × 50 mm L, 5 μm | 35°C | 210-400 nm |
| E | Nexera UC LCMS-2020 | Ascentis Express C18 2.1 mm I.D. × 50 mm L, 2.7 μm | 35°C | 210-400 nm |

[Table 3-2]

| Table 3 (cont.) | | | | |
|---|---|---|---|---|
| Analysis conditions | Mobile phase | Gradient | | Flow rate (mL/min) |
| | | Time after injection (min) | A/B | |
| A | A) 0.05% TFA/ $H_2O$<br>B) 0.05% TFA/ MeCN | 0-5.0<br>5.0-6.0 | 100/0 → 0/100<br>0/100 | 0.5 |
| B | A) 0.1% FA/ MeCN<br>B) 0.1% FA/ $H_2O$ | 0-1.0<br>1.0-1.4 | 5/95 → 100/0<br>100/0 | 1 |
| C | A) 0.05% TFA/ $H_2O$<br>B) 0.05% TFA/ MeCN | 0-10.0<br>10.0-15.0<br>15.0-16.0 | 65/35 → 55/45<br>55/45 → 0/100<br>0/100 | 0.5 |
| D | A) MeOH<br>B) 10 mM AA/ $H_2O$ | 0-1.0<br>1.0-1.4 | 5/95 → 100/0<br>100/0 | 0.9 |
| E | A) 0.05% TFA/ MeCN<br>B) 0.05% TFA/ $H_2O$ | 0-1.5<br>1.5-2.0 | 5/95 → 100/0<br>100/0 | 1 |

NMR was performed using a JEOL JNM-ECZ500R Nuclear Magnetic Resonance spectrometer. The NMR data were shown in ppm (parts per million) (δ) and the deuterium lock signal from the sample solvent was used as a reference.
Commercially available reagents were used directly without further purification.
All of the nonaqueous reactions were conducted in anhydrous solvents.
Concentration under reduced pressure and solvent distillation were carried out using a rotary evaporator.

(Synthetic Example 1)

Synthesis of (2-amino-3-fluoropyridin-4-yl)methyl methyl carbonate (compound 4A)

[Chemical Formula 12]

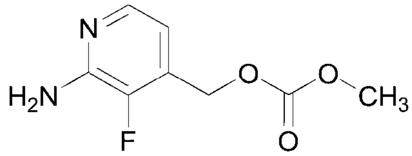

(1-1) Synthesis of (2-amino-3-fluoropyridin-4-yl)methyl methyl carbonate (compound 4A)

(2-Amino-3-fluoropyridin-4-yl)methanol (compound 2A) (30.0 g, 211 mmol) and DMAP (28.4 g, 232 mmol) were added to a reaction vessel, and then this was purged with nitrogen. MeCN (885 mL) was added, and after confirming that the reaction solution had been homogenized, methyl chloroformate (compound 3A) (16.2 mL, 211 mmol) was added dropwise over 1 hour. MeCN (15 mL) was added and the solution was stirred at 25°C for 2 hours. After distilling off the solvent at 40°C under reduced pressure to give a solution volume of 90 mL, isopropyl acetate (900 mL) was added. This solution was washed once with a 15% sodium chloride aqueous solution (300 mL), once with a 15% ammonium chloride aqueous solution (300 mL), and twice with water (150 mL), and then the solvent was distilled off at 40°C under reduced pressure to give a solution volume of 90 mL. Toluene (300 mL) was added to the resulting solution, and then the solvent was distilled off at 40°C under reduced pressure to give a solution volume of 90 mL. Toluene (300 mL) was again added, and the solvent was distilled off at 40°C under reduced pressure to give a solution volume of 90 mL. Toluene (30 mL) was further added and the internal temperature of the solution was increased to 60°C to dissolve the precipitated solid, and then the temperature was decreased to 40°C over 30 minutes. Seed crystals (75 mg) obtained in (1-2) below were added and the solution was stirred for 30 minutes. Then the internal temperature of the solution was decreased to 25°C over 30 minutes and the solution was stirred for 30 minutes. n-Heptane (60 mL) was added over 30 minutes and the solution was stirred for 30 minutes. n-Heptane (60 mL) was again added over 30 minutes and the solution was stirred for 1 hour and 30 minutes. n-Heptane (120 mL) was further added over 30 minutes and the solution was stirred for 30 minutes. The precipitated solid was filtered off and washed with a mixed solvent of n-heptane (45 mL) and toluene (15 mL), and then dried under reduced pressure to give compound 4A (35.1 g, 83% yield).

HPLC purity: 98.86% (analysis conditions A)
$^1$H-NMR (DMSO-$d_6$) δ: 7.69 (1H, d, J = 5.2 Hz), 6.47 (1H, t, J = 4.9 Hz), 6.26 (2H, s), 5.15 (2H, s), 3.73 (3H, s).
MS ($ESI^+$) m/z: 201 $[M+H]^+$

(1-2) Synthesis of seed crystals of (2-amino-3-fluoropyridin-4-yl)methyl methyl carbonate (compound 4A)

(2-Amino-3-fluoropyridin-4-yl)methanol (compound 2A) (10.0 g, 70.4 mmol) and DMAP (12.9 g, 106 mmol) were added to a reaction vessel under a nitrogen atmosphere, and then purging with nitrogen was performed. MeCN (300 mL) was added, the mixture was stirred, and methyl chloroformate (compound 3A) (5.4 mL, 70 mmol) was added dropwise to the resulting homogeneous solution at an external temperature of 25°C over 1 hour. The resulting reaction solution was stirred for 3 hours and then concentrated under reduced pressure. 2-MeTHF (100 mL) was added to the concentrated residue and concentrated under reduced pressure, and 2-MeTHF (100 mL) was again added to the residue and concentrated. After adding 2-MeTHF (50 mL) to the concentrated residue, the precipitated solid was filtered off and the filtration residue was washed with 2-MeTHF (30 mL) (the resulting wash solution being referred to as the wash solution 1). The remaining filtration residue was again washed with 2-MeTHF (100 mL) (the resulting wash solution being referred to as the wash solution 2). The filtrate and the wash solution 1 were combined and the mixture was concentrated under reduced pressure to a total volume of 25 mL. Then 2-MeTHF (5 mL) was added, and the mixture was heated and stirred at an external temperature of 40°C to give a homogeneous solution. n-Heptane (30 mL) was added to this solution over 1 hour, and this was cooled to an external temperature of 35°C. n-Heptane (30 mL) was again added to the resulting mixture over 1 hour, and the mixture was cooled to an external temperature of 25°C. The precipitated solid was filtered off and the filtration residue was washed with a liquid mixture of n-heptane/2-MeTHF (3:1, 28 mL). The filtrate and the wash solution were combined and the mixture was concentrated under reduced pressure. The concentrated residue, the residue remaining in the reaction vessel after filtration, and the residue obtained by concentrating the wash solution 2 under reduced pressure were combined, and the mixture was purified by silica gel column chromatography using ethyl acetate and n-heptane as the mobile phase. The resulting solution of compound 4A in ethyl acetate/ n-heptane was concentrated under reduced pressure. The colorless solid obtained by the concentration was dried at an external temperature of 40°C under reduced pressure to give compound 4A (4.2 g, 30% yield) as crystals.
HPLC purity: 99.99% (analysis conditions A)

(Synthetic Example 2)

Synthesis of 5-bromo-2-(4-cyclopropyl-2-fluoroanilino)-3,4-difluorobenzamide (compound 9A)

[Chemical Formula 13]

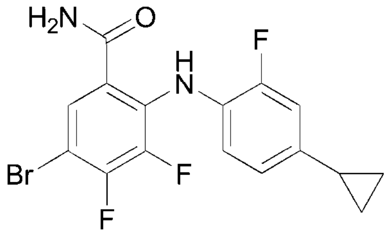

(2-1) Synthesis of 5-bromo-2-(4-cyclopropyl-2-fluoroanilino)-3,4-difluorobenzoic acid (compound 8A)

[Chemical Formula 14]

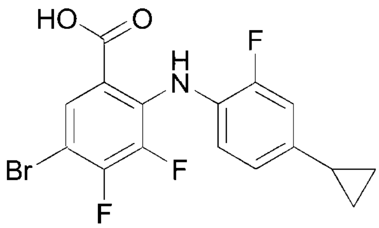

A reaction vessel in which a 1 M lithium bis(trimethylsilyl)amide THF solution (206 mL, 206 mmol) had been placed was cooled to an external temperature of -15°C, and a solution of 4-cyclopropyl-2-fluoroaniline (11.6 g, 76.5 mmol) in THF (30 mL) was added dropwise. Then a solution of 5-bromo-2,3,4-trifluorobenzoic acid (15.0 g, 58.8 mmol) in THF (120 mL) was added dropwise over 30 minutes and the mixture was stirred for 30 minutes. Then 5 M hydrochloric acid (118 mL) was added to the reaction mixture, the temperature was increased to room temperature, and extraction was performed with isopropyl acetate (75 mL). The organic layer was washed sequentially twice with water (75 mL) and once with a 15% sodium chloride aqueous solution (75 mL), and concentrated under reduced pressure. Acetone (120 mL) was added to the resulting concentrated residue, and after heating to dissolution, water (45 mL) and seed crystals (150 mg) were added to precipitate crystals. Water (45 mL) was added to the resulting slurry, and the crystals were filtered off. After washing with a liquid mixture of acetone/water (1/2), they were dried at an external temperature of 40°C under reduced pressure to give compound 8A (19.4 g, 85% yield).

LCMS m/z: 386 $[M+H]^+$
HPLC retention time: 0.62 min (analysis conditions B)

(2-2) Synthesis of 5-bromo-2-(4-cyclopropyl-2-fluoroanilino)-3,4-difluorobenzamide (compound 9A)

MeCN (104 mL), THF (26 mL), and 1,1'-carbonyldiimidazole (8.2 g, 50.5 mmol) were added to a reaction vessel in which 5-bromo-2-(4-cyclopropyl-2-fluoroanilino)-3,4-difluorobenzoic acid (compound 8A) (13.0 g, 33.7 mmol) had been placed, and the mixture was stirred for 2 hours at room temperature. After adding 28% ammonia water (13 mL) to the reaction mixture, this was stirred for 30 minutes at room temperature, and then water (117 mL) was added over 1 hour. Crystals were filtered off and washed with water, and then dried at an external temperature of 40°C under reduced pressure to give compound 9A (12.0 g, 93% yield).

LCMS m/z: 385 $[M+H]^+$
HPLC retention time: 0.52 min (analysis conditions B)

(Synthetic Example 3)

Synthesis of 5-[(2-amino-3-fluoropyridin-4-yl)methyl]-2-(4-cyclopropyl-2-fluoroanilino)-3,4-difluorobenzamide (compound 5A)

[Chemical Formula 15]

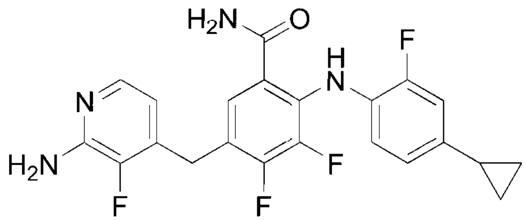

(3-1) Synthesis of 5-[(2-amino-3-fluoropyridin-4-yl)methyl]-2-(4-cyclopropyl-2-fluoroanilino)-3,4-difluorobenzamide (compound 5A)

5-Bromo-2-(4-cyclopropyl-2-fluoroanilino)-3,4-difluorobenzamide (compound 9A) (15.00 g, 38.9 mmol), potassium acetate (11.47 g, 117 mmol), bispinacoldiboron (10.88 g, 42.8 mmol), and 2-MeTHF (113 mL) were added to a reaction vessel, and then the internal area of the reaction vessel was purged with nitrogen. XPhos-Pd-G3 (659 mg, 0.779 mmol) was added to the reaction solution, and then the internal area of the reaction vessel was purged with nitrogen. The internal temperature of the reaction solution was increased to 80°C and the solution was stirred for 4 hours. The internal temperature of the reaction solution was decreased to 25°C, and then (2-amino-3-fluoropyridin-4-yl)methyl methyl carbonate (compound 4A) (11.69 g, 58.4 mmol), potassium carbonate (16.15 g, 117 mmol), and RuPhos-Pd-G3 (1.629 g, 1.947 mmol) were added. The internal area of the reaction vessel was purged with nitrogen (the oxygen concentration in the reaction vessel ≤ 0.1%), and then the internal temperature of the reaction solution was increased to 75°C. EtOH (52 mL, 900 mmol) was slowly added dropwise (so that the internal temperature of the solution did not fall below 60°C), and then the solution was stirred for 4 hours and 30 minutes. After confirming degradation of compound 4A (less than 1%), 2-MeTHF (113 mL) and an N-acetyl-L-cysteine aqueous solution (1.271 g, 113 mL) were added and the mixture was stirred for 1 hour. The internal temperature of the reaction solution was decreased to 40°C, and then extraction was performed. The organic layer was washed sequentially with 0.1 M hydrochloric acid (113 mL), a 0.1 M potassium phosphate aqueous solution (113 mL), and a 2% sodium chloride aqueous solution (113 mL), and the resulting organic layer was filtered. The filtrate was concentrated under reduced pressure to a total volume of 75 mL, and toluene (225 mL) was added to the concentrated residue. The solvent was distilled off under reduced pressure to give a solution volume of 75 mL, and then toluene (225 mL) was again added. The solvent was distilled off under reduced pressure to give a solution volume of 75 mL, and then 1-butanol (12 mL) was added as an internal standard. $^1$H-NMR was used to determine the content of toluene, and toluene was added to give a solution containing 240 mL of toluene. The internal temperature of the reaction solution was increased to 110°C, and after confirming complete dissolution of solids, it was decreased to 90°C. Seed crystals (75 mg) obtained in (4-2) (5) below were added, and then the internal temperature of the reaction solution was decreased to 80°C and the mixture was stirred for 1 hour. The temperature of the solution was decreased to 60°C and the solution was stirred for 30 minutes. The temperature of the solution was decreased to 40°C and the solution was stirred for 30 minutes. The temperature of the solution was decreased to 25°C and the solution was stirred for 30 minutes. The temperature of the solution was decreased to 5°C and the solution was stirred for 30 minutes. The precipitated solid was filtered off, washed with toluene (45 mL), and filtered under reduced pressure to give compound 5A (12.6 g, 75% yield).

HPLC purity: 99.69% (analysis conditions A)
$^1$H-NMR (DMSO-$d_6$) δ: 9.38 (1H, brs), 8.19 (1H, brs), 7.70 (1H, brs), 7.65 (1H, d, J = 5.2 Hz), 7.58 (1H, d, J = 6.9 Hz), 6.90 (1H, d, J = 13.2), 6.81-6.76 (2H, m), 6.38 (1H, t, J = 5.2 Hz), 6.14 (2H, brs), 3.91 (2H, s), 1.90-1.84 (1H, m), 0.92-0.88 (2H, m), 0.64-0.61 (2H, m).
MS (ESI$^+$) m/z: 431 [M+H]$^+$

(Synthetic Example 4)

Synthesis of a sodium salt of 2-(4-cyclopropyl-2-fluoroanilino)-3,4-difluoro-5-[[3-fluoro-2-(methylsulfamoylamino)pyridin-4-yl]methyl]benzamide (compound 1A)

[Chemical Formula 16]

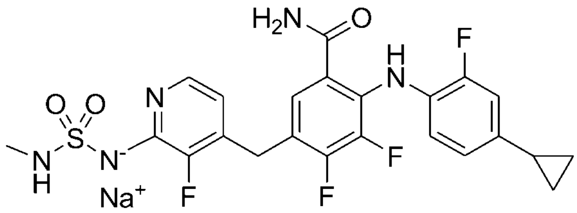

(4-1) Preparation of sample 1a (Form I)

5-[(2-Amino-3-fluoropyridin-4-yl)methyl]-2-(4-cyclopropyl-2-fluoroanilino)-3,4-difluorobenzamide (compound 5A) (3.00 g, 6.97 mmol) was added to a reaction vessel, and then 1,3-dimethyl-2-imidazolidinone (12 mL) and THF (6 mL) were added to dissolve it. After purging the reaction vessel with nitrogen, pyridine (1.69 mL, 20.91 mmol) was added and the mixture was cooled to 0°C. N-Methylsulfamoyl chloride (0.67 mL, 7.67 mmol) was added and the mixture was stirred for 45 minutes, and then pyridine (0.10 mL, 1.26 mmol) and N-methylsulfamoyl chloride (0.30 mL, 3.42 mmol) were added and the mixture was stirred for 35 minutes. Subsequently, pyridine (0.24 mL, 2.93 mmol) and N-methylsulfamoyl chloride (0.13 mL, 1.46 mmol) were added and the mixture was stirred for 35 minutes, and then pyridine (0.09 mL, 1.12 mmol) and N-methylsulfamoyl chloride (0.05 mL, 0.56 mmol) were added and the mixture was stirred for 3 hours. The reaction solution was diluted with THF (18 mL) and TBME (24 mL), and then a 10% sodium chloride aqueous solution (15 g) was added to stop the reaction, after which the temperature was increased to 25°C. The reaction solution was separated into two layers, and the upper layer (organic layer) was washed with a 10% sodium chloride aqueous solution (24 g). The resulting organic layer was concentrated under reduced pressure to 15 mL and diluted with THF (45 mL). This procedure was repeated two more times, and then the precipitated inorganic salts were filtered off. The filtered off inorganic salts were washed with THF (15 mL) and this was combined with the filtrate. Then the mixture was concentrated under reduced pressure to 15 mL. The residue was diluted with acetone (11 mL), and then THF (9.3 mL) was added. After warming the resulting solution to 40°C, a 5M sodium hydroxide aqueous solution (1.32 mL, 6.62 mmol) and a suspension of seed crystals of a sodium salt of compound 1A (1.82 mg) (sample 1b mentioned below) in acetone (0.7 mL) were sequentially added and the mixture was stirred for 2 hours and 30 minutes. Acetone (6.6 mL) was added over 30 minutes and the mixture was stirred for 2 hours. Acetone (18.2 mL) was added over 20 minutes and the mixture was stirred for 45 minutes. TBME (24 mL) was added over 20 minutes and the mixture was stirred for 50 minutes. The resulting suspension was cooled to 25°C over 30 minutes, stirred for 1 hour, and allowed to stand at room temperature overnight. After standing overnight, the suspension was stirred at 25°C for 2 hours and 30 minutes. The precipitated solid was filtered off, washed with a mixed solvent of acetone (11.0 mL) and TBME (11.0 mL), and dried under reduced pressure to give a sodium salt of compound 1A (2.77 g, 73% yield) (sample 1a (Form I)).

HPLC purity: 99.49% (analysis conditions C)
HPLC retention time: 7.02 min (analysis conditions C)
$^1$H-NMR (DMSO-$d_6$) $\delta$: 9.36 (1H, brs), 8.21 (1H, brs), 7.68 (1H, brs), 7.60-7.55 (2H, m), 6.89 (1H, brd, J = 13.5 Hz), 6.82-6.75 (2H, m), 6.17 (1H, t, J = 5.0 Hz), 5.50 (1H, q, J = 6.0 Hz), 3.85 (2H, s), 2.28 (3H, d, J = 6.0 Hz), 1.90-1.83 (1H, m), 0.92-0.87 (2H, m), 0.64-0.60 (2H, m).
MS (ESI$^+$) m/z: 524 $[M+2H-Na]^+$

(4-2) Preparation of sample 1b (Form I)

(1) Synthesis of N-[4-[[*tert*-butyl(dimethyl)silyl]oxymethyl]-3-fluoropyridin-2-yl]acetamide

*tert*-Butyl-[(2-chloro-3-fluoropyridin-4-yl)methoxy]-dimethylsilane (180 g, 653 mmol), Xantphos (22.7 g, 39.2 mmol), potassium carbonate (135 g, 979 mmol), acetamide (77.1 g, 1.31 mol), and 2-methyl-2-butanol (540 mL) were added to a reaction vessel, and then this was deaerated under reduced pressure and purged with nitrogen. After then adding tris(dibenzylideneacetone)dipalladium(0) (14.9 g, 16.3 mmol) and toluene (540 mL), deaeration under reduced pressure and purging with nitrogen were further performed. The mixture was warmed to an external temperature of 120°C under a nitrogen atmosphere and stirred for 7 hours. The external temperature was cooled to room temperature, and the reaction mixture was filtered and washed with toluene (450 mL). Active carbon (9.00 g, 749 mmol) was added to the filtrate and the mixture was stirred for 1 hour at room temperature. The reaction mixture was then filtered and washed twice with toluene (270 mL the first time and 180 mL the second time) to give a crude product of N-[4-[[*tert*-butyl(dimethyl)silyl]oxymethyl]-3-fluoropyridin-2-yl]acetamide as a toluene solution.

LCMS m/z: 299 [M+H]+
HPLC retention time: 0.81 min (analysis conditions B)

(2) Synthesis of N-[3-fluoro-4-(hydroxymethyl)pyridin-2-yl]acetamide methanesulfonate (compound 13A)

[Chemical Formula 17]

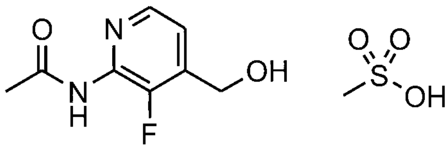

The resulting toluene solution of N-[4-[[*tert*-butyl(dimethyl)silyl]oxymethyl]-3-fluoropyridin-2-yl]acetamide, toluene (175 mL), and MeOH (195 mL) were added to a reaction vessel, and this was deaerated under reduced pressure and purged with nitrogen. Methanesulfonic acid (188 g, 1.96 mol) was added dropwise at an external temperature of 10°C and the mixture was stirred for 2 hours at room temperature. The reaction mixture was cooled to an external temperature of 0°C and stirred for 3 hours. The precipitate was filtered off and washed with a cooled liquid mixture of toluene (312 mL) and MeOH (78 mL). The filtered off solid and a liquid mixture of toluene (1.1 L) and EtOH (492 mL) were added to a reaction vessel and stirred at an external temperature of 0°C for 1 hour. The solid was filtered off, washed with a liquid mixture of toluene (281 mL) and EtOH (117 mL), and dried at an external temperature of 40°C under reduced pressure to give compound 13A (149 g, 81% yield).

LCMS m/z: 185 [M+H]+
HPLC retention time: 0.30 min (analysis conditions D)

(3) Synthesis of (2-acetamide-3-fluoropyridin-4-yl)methyl methyl carbonate (compound 14A)

[Chemical Formula 18]

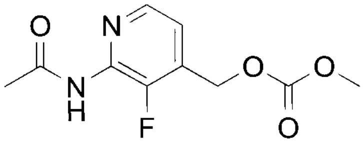

DMAP (52.3 g, 428 mmol) was added at room temperature to a reaction vessel in which N-[3-fluoro-4-(hydroxymethyl)pyridin-2-yl]acetamide methanesulfonate (compound 13A) (50.0 g, 178 mmol) and 2-MeTHF (750 mL) had been placed. The external temperature was cooled to 0°C and methyl chloroformate (21.9 g, 232 mmol) was added. Then the temperature was increased to room temperature and the mixture was stirred. The precipitated solid was filtered off and the filtrate was concentrated under reduced pressure at an external temperature of 40°C. Ethyl acetate (300 mL) was added to the concentrated residue to dissolve it at room temperature, and then DIPEA (31.2 mL, 178 mmol), n-heptane (150 mL) and seed crystals were added. After confirming precipitation of crystals, n-heptane (1 L) was added. The slurry was cooled to an external temperature of 0°C, and then the crystals were filtered off and washed with a liquid mixture of ethyl acetate/ n-heptane (2/7). They were dried at an external temperature of 40°C under reduced pressure to give compound 14A (31.3 g, 72% yield) as a colorless solid.

LCMS m/z: 243 [M+H]+
HPLC retention time: 0.37 min (analysis conditions B)

(4) Synthesis of 5-[(2-acetamide-3-fluoropyridin-4-yl)methyl]-2-(4-cyclopropyl-2-fluoroanilino)-3,4-difluorobenzamide (compound 15A)

[Chemical Formula 19]

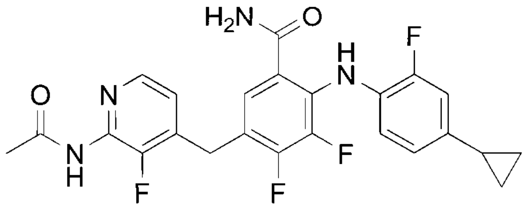

5-Bromo-2-(4-cyclopropyl-2-fluoroanilino)-3,4-difluorobenzamide (compound 9A) (10.0 g, 26.0 mmol), bis(pinacolato)diboron (7.3 g, 28.6 mmol), potassium acetate (7.6 g, 77.9 mmol), and 2-MeTHF (150 mL) were added to a reaction vessel, and this was deaerated under reduced pressure and purged with nitrogen. (2-Dicyclohexylphosphino-2',4',6'-triisopropyl-1,1'-biphenyl)[2-(2'-amino-1,1'-biphenyl)]palladium(II) methanesulfonate (440 mg, 0.52 mmol) was added, and deaeration under reduced pressure and purging with nitrogen were further performed. The mixture was warmed to an external temperature of 80°C under a nitrogen atmosphere and stirred for 6 hours. The external temperature was cooled to room temperature, potassium carbonate (10.8 g, 77.9 mmol) was added, and deaeration under reduced pressure and purging with nitrogen were performed. (2-Dicyclohexylphosphino-2',4',6'-triisopropyl-1,1'-biphenyl)[2-(2'-amino-1,1'-biphenyl)]palladium(II) methanesulfonate (1.1 g, 1.3 mmol) was added, and after deaeration under reduced pressure and purging with nitrogen, a solution of (2-acetamide-3-fluoropyridin-4-yl)methyl methyl carbonate (compound 14A) (12.6 g, 51.9 mmol) in 2-MeTHF (150 mL) was added. The mixture was warmed to an external temperature of 70°C under a nitrogen atmosphere, and after adding water (935 μL, 51.9 mmol) three times at 20 minute intervals, the mixture was stirred for 20 minutes. Water (7.0 mL) was further added dropwise, the mixture was stirred for 2 hours, a solution prepared from N-acetylcysteine (847 mg, 5.2 mmol) and water (150 mL) was added, and the mixture was stirred for 1 hour. After cooling to an external temperature of 40°C, the aqueous layer was discharged. The organic layer was washed with a 15% sodium chloride aqueous solution (150 mL), the insoluble portion was filtered, and concentration was carried out under reduced pressure. MeCN (500 mL) was added to the resulting concentrated residue and heated to an external temperature of 100°C to dissolve it, and then the temperature was cooled to room temperature. Crystals were filtered off, washed with MeCN (200 mL), and dried at an external temperature of 40°C under reduced pressure to give compound 15A (8.34 g, 68% yield).

LCMS m/z: 471 $[M-H]^-$
HPLC retention time: 0.74 min (analysis conditions B)

(5) Synthesis of 5-[(2-amino-3-fluoropyridin-4-yl)methyl]-2-(4-cyclopropyl-2-fluoroanilino)-3,4-difluorobenzamide (compound 5A)

[Chemical Formula 20]

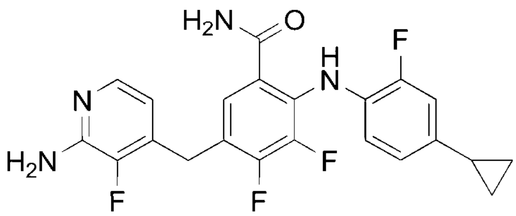

MeOH (3 mL) and 5M hydrochloric acid (0.42 mL, 2.1 mmol) were added to a reaction vessel in which 5-[(2-acetamide-3-fluoropyridin-4-yl)methyl]-2-(4-cyclopropyl-2-fluoroanilino)-3,4-difluorobenzamide (compound 15A) (100 mg, 0.21 mmol) had been placed, and the mixture was stirred at an external temperature of 50°C for 6 hours. The reaction mixture was cooled to room temperature, and a 2 M sodium hydroxide aqueous solution (1.1 mL, 2.1 mmol) was added. Water (0.5 mL) was added to the resulting slurry, and crystals were filtered off. They were washed with a liquid mixture of MeOH/water (3/2) and dried at an external temperature of 40°C under reduced pressure to give compound 5A (77.7 mg, 85% yield) as a colorless solid.

LCMS m/z: 431 $[M+H]^+$
HPLC retention time: 0.61 min (analysis conditions B)

(6) Synthesis of 2-(4-cyclopropyl-2-fluoroanilino)-3,4-difluoro-5-[[3-fluoro-2-(methylsulfamoylamino)pyridin-4-yl]methyl]benzamide (compound 1A)

[Chemical Formula 21]

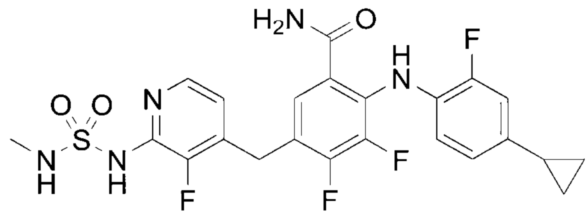

5-[(2-Amino-3-fluoropyridin-4-yl)methyl]-2-(4-cyclopropyl-2-fluoroanilino)-3,4-difluorobenzamide (compound 5A) (100 mg, 0.232 mmol) was dissolved in anhydrous DMA (1 mL), and pyridine (56.4 μL, 0.697 mmol) was added. After then cooling to 0°C, methylsulfamoyl chloride (30.2 μL, 0.349 mmol) was added and the mixture was stirred for 1 hour. MeCN (0.6 mL), water (0.3 mL), and seed crystals (which had been obtained in Production Example 1A-1 below) (1 mg) were added to the reaction mixture, the temperature was increased to room temperature, water (0.7 mL) and MeCN (0.4 mL) were added, and the mixture was stirred for 20 hours. The precipitate was filtered off and washed with a liquid mixture of MeCN/water (1/1) to give compound 1A (93.1 mg, 77% yield) as a colorless solid.

LCMS m/z: 524 $[M+H]^+$
HPLC retention time: 1.13 min (analysis conditions E)

(7) Preparation of a sodium salt of 2-(4-cyclopropyl-2-fluoroanilino)-3,4-difluoro-5-[[3-fluoro-2-(methylsulfamoylamino)pyridin-4-yl]methyl]benzamide (compound 1A)

(i) Preparation of sample 1b (Form I)

Acetone (10.6 mL) and DMSO (1.51 mL) were added to 2-(4-cyclopropyl-2-fluoroanilino)-3,4-difluoro-5-[[3-fluoro-2-(methylsulfamoylamino)pyridin-4-yl]methyl]benzamide (compound 1A) (3.03 g), and this was dissolved at room temperature. A 20% sodium ethoxide EtOH solution (3.03 mL) and seed crystals of a sodium salt of compound 1A (sample 1c mentioned below) were added to the solution, and the mixture was stirred at room temperature for 1 hour. Then EtOH (15.1 mL) was added and the mixture was stirred at room temperature for 4 hours. Then EtOH (15.1 mL) was further added, and the mixture was stirred at room temperature for 4 hours to give a sodium salt of compound 1A (2.74 g) as powdered crystals (sample 1b (Form I)).

(ii) Preparation of sample 1c

A 20% sodium ethoxide EtOH solution (0.054 mL) and methyl isobutyl ketone (0.161 mL) were added to compound 1A (53.6 mg), and the mixture was stirred at room temperature for 30 minutes. Then methyl isobutyl ketone (0.161 mL) was added and the mixture was stirred at 60°C for 4 days. Then DMSO (0.054 mL) was added, and the mixture was stirred at 60°C for 5 hours to give a sodium salt of compound 1A (25.6 mg) as powdered crystals (sample 1c).

(4-3) Powder X-ray diffraction analysis

Sample 1a (Form I) was subjected to powder X-ray diffraction analysis under the following conditions.

Apparatus: Empyrean (PANalytical)
Anti-cathode: Cu
Tube voltage: 45 kV
Tube current: 40 mA
Scan mode: Continuous
Step width: 0.0262606°
Scan axis: 2θ
Sampling time per step: 5.100 seconds

Scan range: 3° to 25°

Sample 1b (Form I) and sample 1c were subjected to powder X-ray diffraction analysis under the following conditions.

Apparatus: SmartLab, D/Tex Ultra detector (Rigaku Corp.)
Anti-cathode: Cu
Tube voltage: 45 kV
Tube current: 200 mA
Sampling width: 0.02°

The results of powder X-ray diffraction analysis are shown in Figs. 1 to 3. Fig. 1 shows a powder X-ray diffraction pattern of sample 1a (Form I). Fig. 2 shows a powder X-ray diffraction pattern of sample 1b (Form I). Fig. 3 shows a powder X-ray diffraction pattern of sample 1c. In Figs. 1 to 3, the horizontal axis (X-axis) represents the diffraction angle 2θ (°) and the vertical axis (Y-axis) represents the diffraction intensity.

(4-4) Ion chromatography

The proportion of sodium ions in the crystal was measured for sample 1b (Form I) by ion chromatography, and the molar ratio of sodium ions to compound 1A was 0.99. From this, it was confirmed that sample 1b is a monosodium salt. The ion chromatography was carried out under the conditions below.

Apparatus: Dionex ICS-1600, AS-AP (manufactured by Thermo Fisher Scientific)

Column: Dionex IonPac CG16 (5 × 50 mm)/CS16 (5 × 250 mm) (manufactured by Thermo Fisher Scientific)

Eluent: 30 mmol/L methanesulfonic acid solution
Suppressor: Dionex CERS-500 4 mm, 88 mA (manufactured by Thermo Fisher Scientific)
Column temperature: 40°C
Eluent flow rate: 1.00 mL/min
Sample injection volume: 10 μL
Detector: conductivity detector

Sample treatment: Sample 1b was suspended in a 20 mmol/L methanesulfonic acid solution at a concentration of 0.5 mg/mL, shaken and stirred for 17 hours to extract sodium ions, and the supernatant was measured.

[Production Examples]

In the production examples below, NMR analysis was conducted using an AVANCE III HD400 (400 MHz) by Bruker Co. The NMR data were shown in ppm (parts per million) (δ) and the deuterium lock signal from the sample solvent was used as a reference.

The mass spectrum data were obtained using an ultra-high performance liquid chromatography (Nexera UC)-equipped single quadrupole mass spectrometer (LCMS-2020) by Shimadzu Corp. or an Acquity ultra-high performance liquid chromatography (UPLCor UPLC I-Class)-equipped single quadrupole mass spectrometer (SQD or SQD2) by Waters Co.

High-performance liquid chromatography (HPLC) analysis was conducted using one of the analysis conditions listed in Table 4 below.

[Table 4-1]

Table 4

| Analysis conditions | Apparatus | Column | Column temperature | Detection wavelength (PDA) |
|---|---|---|---|---|
| E | Nexera UC<br>LCMS-2020 | Ascentis Express C18<br>2.1 mm I.D. × 50 mm L, 2.7 μm | 35°C | 210-400 nm |

(continued)

| Table 4 | | | | |
|---|---|---|---|---|
| Analysis conditions | Apparatus | Column | Column temperature | Detection wavelength (PDA) |
| B | Acquity SQD/SQD2 | Ascentis Express C18 2.1 mm I.D. × 50 mm L, 2.7 μm | 35°C | 210-400 nm |
| I | Acquity SQD/SQD2 | Ascentis Express C18 2.1 mm I.D. × 50 mm L, 2.7 μm | 35°C | 210-400 nm |

[Table 4-2]

| Table 4 (cont.) | | | | |
|---|---|---|---|---|
| Analysis conditions | Mobile phase | Gradient | | Flow rate (mL/min) |
| | | Time after injection (min) | A/B | |
| E | A) 0.05% TFA/ MeCN<br>B) 0.05% TFA/ $H_2O$ | 0-1.5<br>1.5-2.0 | 5/95 → 100/0<br>100/0 | 1 |
| B | A) 0.1% FA/ MeCN<br>B) 0.1% FA/ $H_2O$ | 0-1.0<br>1.0-1.4 | 5/95 → 100/0<br>100/0 | 1 |
| I | A) 0.1% FA/ MeCN<br>B) 0.1% FA/ $H_2O$ | 0-1.0<br>1.0-1.4 | 40/60 → 100/0<br>100/0 | 1 |

Commercially available reagents were used directly without further purification.

All of the nonaqueous reactions were conducted in anhydrous solvents.

Concentration under reduced pressure and solvent distillation were carried out using a rotary evaporator.

Compound a1:

Methyl 3,4-difluoro-2-(2-fluoro-4-iodoanilino)-5-formylbenzoate

[Chemical Formula 22]

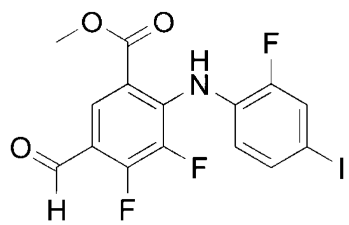

A mixed suspension of 3,4-difluoro-2-((2-fluoro-4-iodophenyl)amino)-5-formylbenzoic acid (5.50 g, 13.1 mmol) in toluene (44 mL) and MeOH (11 mL) was cooled to 0°C, a 10% diazomethyltrimethylsilane hexane solution (21.8 mL, 13.1 mmol) was added, and the mixture was stirred for 64 hours at room temperature. Acetic acid (0.748 mL) was added to the reaction mixture, which was then concentrated under reduced pressure. The resulting residue was purified by trituration (hexane/ethyl acetate) to give the title compound (5.01 g, 88%) as a colorless solid.

LCMS m/z: 436 $[M+H]^+$

HPLC retention time: 1.00 min (analysis conditions 1)

Compound a2:

Methyl 3,4-difluoro-2-(2-fluoro-4-iodoanilino)-5-[(E)-[(4-methylphenyl)sulfonylhydrazinylidene]methyl]benzoate

[Chemical Formula 23]

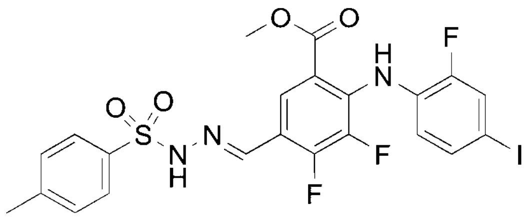

4-Methylbenzenesulfonyl hydrazide (2.14 g, 11.5 mmol) was added to a suspension of methyl 3,4-difluoro-2-(2-fluoro-4-iodoanilino)-5-formylbenzoate (compound a1, 5.00 g, 11.5 mmol) in EtOH (100 mL), and the mixture was stirred for 3 hours at room temperature. The reaction mixture was concentrated under reduced pressure, and then hexane (150 mL) was added. The mixture was cooled to 0°C and filtered, and then washed with hexane (30 mL) to give the title compound (7.05 g, quant.) as a solid.

LCMS m/z: 604 $[M+H]^+$
HPLC retention time: 1.06 min (analysis conditions I)

Compound a3:

N-(2,4-Dimethoxybenzyl)-3-fluoro-4-iodopyridine-2-amine

[Chemical Formula 24]

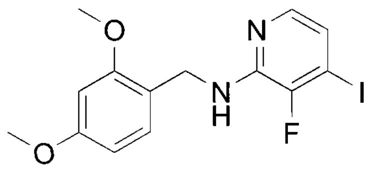

Triethylamine (3.63 mL, 26.0 mmol) and 1-(2,4-dimethoxyphenyl)methaneamine (3.26 mL, 21.7 mmol) were added to a solution of 2,3-difluoro-4-iodopyridine (2.09 g, 8.67 mmol) in NMP (32 mL), and the mixture was stirred for 1.5 hours at 100°C. Water was added to the reaction mixture, and extraction was performed with ethyl acetate. The organic layer was washed with 13% brine, dried over anhydrous sodium sulfate and, after filtering off the drying agent, concentrated under reduced pressure. The resulting residue was purified by silica gel column chromatography (hexane/ethyl acetate) to give the title compound (3.20 g, 95%) as an oil.

LCMS m/z: 389 $[M+H]^+$
HPLC retention time: 0.94 min (analysis conditions B)

Compound a4:

[2-[(2,4-Dimethoxyphenyl)methylamino]-3-fluoropyridin-4-yl]boronic acid

[Chemical Formula 25]

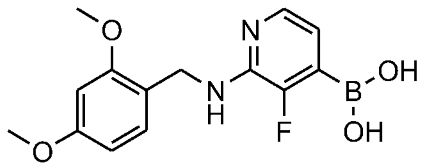

A 1,4-dioxane solution (27 mL) of N-(2,4-dimethoxybenzyl)-3-fluoro-4-iodopyridine-2-amine (compound a3, 2.70 g, 6.96 mmol), [1,1-bis(diphenylphosphino)ferrocene]dichloropalladium(II) dichloromethane addition product (568 mg, 0.696 mmol), potassium acetate (2.05 g, 20.9 mmol) and bis(pinacolato)diboron (2.65 g, 10.4 mmol) was stirred under a nitrogen atmosphere for 5 hours at 90°C and then for 19 hours at 110°C. The reaction mixture was concentrated under reduced pressure, and the resulting residue was purified by reversed-phase column chromatography (0.1% formic acid aqueous solution/ 0.1% formic acid acetonitrile solution) to give the title compound (2.07 g, 97%) as an oil.

LCMS m/z: 307 $[M+H]^+$
HPLC retention time: 0.44 min (analysis conditions B)

Compound a5:

Methyl 5-[[2-[(2,4-dimethoxyphenyl)methylamino]-3-fluoropyridin-4-yl]methyl]-3,4-difluoro-2-(2-fluoro-4-iodoanilino) benzoate

[Chemical Formula 26]

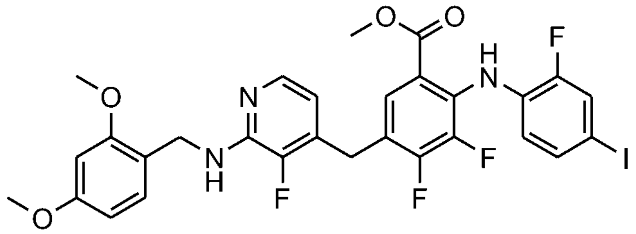

A 1,4-dioxane suspension (59 mL) of methyl 3,4-difluoro-2-(2-fluoro-4-iodoanilino)-5-[(E)-[(4-methylphenyl) sulfonylhydrazinylidene]methyl benzoate (compound a2, 1.30 g, 2.16 mmol), [2-[(2,4-dimethoxyphenyl)methylamino]-3-fluoropyridin-4-yl]boronic acid (compound a4, 1.98 g, 6.46 mmol) and potassium carbonate (357 mg, 2.59 mmol) was stirred under a nitrogen atmosphere for 2.5 hours at 100°C and then for 3 hours at 110°C. Ethyl acetate was added to the reaction mixture, which was then washed with water and 13% brine. The organic layer was dried over anhydrous sodium sulfate and, after filtering off the drying agent, concentrated under reduced pressure. The resulting residue was purified by silica gel column chromatography (hexane/ ethyl acetate) to give the title compound (524 mg, 36%) as a foam.

LCMS m/z: 682 $[M+H]^+$
HPLC retention time: 1.03 min (analysis conditions I)

Compound a6:

Methyl 5-[(2-amino-3-fluoropyridin-4-yl)methyll-3,4-difluoro-2-(2-fluoro-4-iodoanilino)benzoate

[Chemical Formula 27]

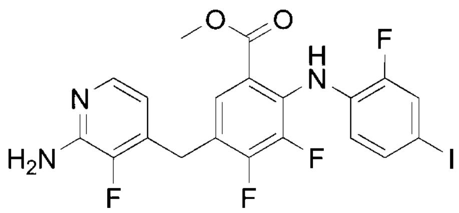

A DCM solution (16 mL) of methyl 5-[[2-[(2,4-dimethoxyphenyl)methylamino]-3-fluoropyridin-4-yl]methyl]-3,4-difluoro-2-(2-fluoro-4-iodoanilino)benzoate (compound a5, 523 mg, 0.768 mmol) was cooled to 0°C, trifluoroacetic acid (15.7 mL) was added, and the mixture was stirred for 1 hour at room temperature. The reaction mixture was concentrated under reduced pressure, and the resulting residue was purified by reversed-phase column chromatography (0.05% trifluoroacetic acid aqueous solution/ 0.05% trifluoroacetic acid acetonitrile solution) to give the title compound (321 mg, 79%) as an oil.

LCMS m/z: 532 $[M+H]^+$
HPLC retention time: 0.55 min (analysis conditions I)

Compound a7:

5-((2-Amino-3-fluoropyridin-4-yl)methyl)-3,4-difluoro-2-((2-fluoro-4-iodophenyl)amino)benzoic acid hydrochloride

[Chemical Formula 28]

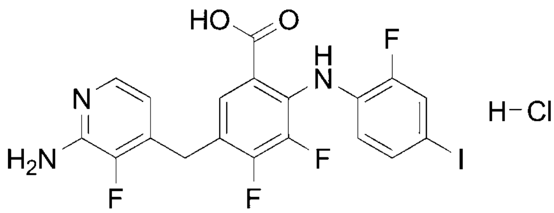

A mixed solution of methyl 5-[(2-amino-3-fluoropyridin-4-yl)methyl]-3,4-difluoro-2-(2-fluoro-4-iodoanilino) benzoate (compound a6, 4.00 g, 7.53 mmol) in THF (64 mL) and water (32 mL) was cooled to 0°C, lithium hydroxide monohydrate (948 mg, 22.6 mmol) was added, and the mixture was stirred for 3.5 hours at room temperature. After cooling to 0°C, 5 M hydrochloric acid (15.1 mL) was added to the reaction mixture, which was then concentrated under reduced pressure. The resulting residue was washed with water and TBME to give the title compound (4.20 g, quant.) as a violet compound.

LCMS m/z: 518 $[M+H]^+$
HPLC retention time: 0.68 min (analysis conditions B)

Compound a8:

5-((2-Amino-3-fluoropyridin-4-yl)methyl)-3,4-difluoro-2-((2-fluoro-4-iodophenyl)amino)benzamide

[Chemical Formula 29]

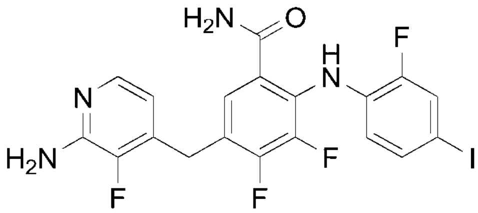

An anhydrous DMF solution (3.6 mL) of 5-((2-amino-3-fluoropyridin-4-yl)methyl)-3,4-difluoro-2-((2-fluoro-4-iodophenyl)amino)benzoic acid hydrochloride (compound a7, 200 mg, 0.361 mmol) was cooled to 0°C, HOOBt (67.8 mg, 0.415 mmol) and EDC·HCl (80.0 mg, 0.415 mmol) were added, and the mixture was stirred for 1.5 hours at room temperature. After further adding HOOBt (8.8 mg, 0.054 mmol) and EDC·HCl (10.4 mg, 0.054 mmol) and stirring at room temperature for 1 hour, a 7 M ammonia MeOH solution (0.103 mL, 0.722 mmol) and DIPEA (0.189 mL, 1.08 mmol) were added at 0°C and the mixture was stirred for 30 minutes at room temperature. Water and a saturated sodium hydrogen carbonate aqueous solution were added at 1:1 to the reaction mixture, and extraction was performed with ethyl acetate. The organic layer was dried over anhydrous sodium sulfate and, after filtering off the drying agent, concentrated under reduced pressure. The resulting residue was dissolved in ethyl acetate (1 mL), and hexane (10 mL) was added. The resulting solid was filtered off and washed with hexane to give the title compound (162 mg, 87%) as a colorless solid.

LCMS m/z: 517 $[M+H]^+$
HPLC retention time: 0.64 min (analysis conditions B)

Compound a9:

5-((2-Amino-3-fluoropyridin-4-yl)methyl)-2-((4-cyclopropyl-2-fluorophenyl)amino)-3,4-difluorobenzamide

[Chemical Formula 30]

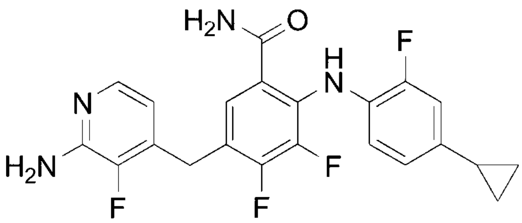

Production Example a9-1:

Tetrakis(triphenylphosphine)palladium(0) (11.2 mg, 9.68 μmol) and 0.5 M cyclopropylzinc bromide (1.94 mL, 0.969 mmol) were added to an anhydrous THF solution (1.9 mL) of 5-((2-amino-3-fluoropyridin-4-yl)methyl)-3,4-difluoro-2-((2-fluoro-4-iodophenyl)amino)benzamide (compound a8, 100 mg, 0.194 mmol), and the mixture was stirred for 2.5 hours at room temperature under a nitrogen atmosphere. Ethyl acetate (5 mL) was added to the reaction mixture, which was then filtered with Celite and washed with ethyl acetate (3 mL). The filtrate was washed with water and saturated brine, and the organic layer was dried over anhydrous sodium sulfate and, after filtering off the drying agent, concentrated under reduced pressure. Dichloromethane/hexane (1/10, 11 mL) was added to the resulting residue, and the solid was filtered off and washed with hexane (3 mL) to give compound a9 (63.4 mg, 76%) as a colorless solid.

LCMS m/z: 431 $[M+H]^+$
HPLC retention time: 0.61 min (analysis conditions B)

Compound r1:

4-Nitrophenyl methylsulfamate

[Chemical Formula 31]

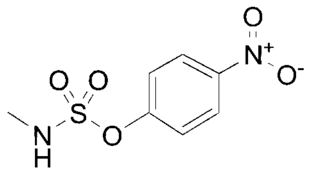

A dichloromethane solution (60 mL) of 4-nitrophenol (5.00 g, 35.9 mmol) and triethylamine (11.3 mL, 81.0 mmol) was cooled to -78°C, a dichloromethane solution (15 mL) of methylsulfamoyl chloride (5.82 g, 44.9 mmol) was added, and the mixture was stirred for 1.5 hours at -78°C. The reaction mixture was concentrated under reduced pressure, and the resulting residue was purified by silica gel column chromatography (hexane/ ethyl acetate) and reversed-phase column chromatography (0.1% formic acid aqueous solution/ 0.1% formic acid acetonitrile solution) to give the title compound (5.51 g, 66%) as a colorless solid.

HPLC retention time: 0.63 min (analysis conditions B)
$^1$H-NMR (400 MHz, $CDCl_3$) δ: 8.31 (2H, m), 7.46 (2H, m), 4.68 (1H, m), 3.00 (3H, d, J = 5.4 Hz).

Compound 1A:

2-(4-Cyclopropyl-2-fluoroanilino)-3,4-difluoro-5-[[3-fluoro-2-(methylsulfamoylamino)pyridin-4-yl]methyl]benzamide

[Chemical Formula 32]

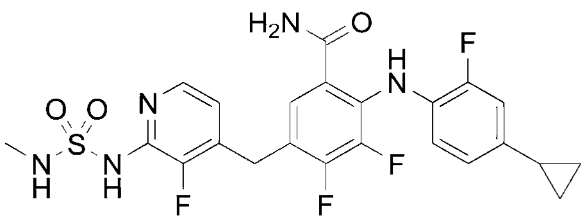

Production Example 1A-1:

After dissolving 5-((2-amino-3-fluoropyridin-4-yl)methyl)-2-((4-cyclopropyl-2-fluorophenyl)amino)-3,4-difluorobenzamide (compound a9, 2.47 g, 5.74 mmol) in anhydrous DMF (28.7 mL), pyridine (2.78 mL, 34.4 mmol) and 4-nitrophenyl methylsulfamate (compound r1, 4.00 g, 17.2 mmol) were added and the mixture was stirred for 2.5 hours at 40°C. The reaction mixture was cooled to room temperature, and water (24.7 mL) was added. After further adding acetonitrile (3 mL) and water (19.8 mL) and stirring for 10 minutes, the solid was filtered off. The resulting solid was washed with water/acetonitrile (1/1, 49.4 mL) to give compound 1A (2.56 g, 85%) as a colorless solid.

LCMS m/z: 524 $[M+H]^+$
HPLC retention time: 1.13 min (analysis conditions E)

## Claims

1. A method for producing a sodium salt of 2-(4-cyclopropyl-2-fluoroanilino)-3,4-difluoro-5-[[3-fluoro-2-(methylsulfamoylamino)pyridin-4-yl]methyl]benzamide (hereinafter, also referred to as "compound 3"), the method comprising step (A-1) below:
(A-1) mixing (a), (b), and (c) below in a first solvent to obtain a mixture containing compound 3:

(a) 2-(4-cyclopropyl-2-fluoroanilino)-3,4-difluoro-5-[[3-fluoro-2-(methylsulfamoylamino)pyridin-4-yl]methyl]benzamide (hereinafter, also referred to as "compound 1");
(b) a sodium base; and
(c) at least one organic acid selected from the group consisting of compounds represented by general formula (A) below:

[Chemical Formula 1]

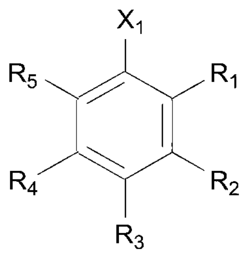

(A)

wherein

$X_1$ is a hydroxy group or a mercapto group; and
$R_1$, $R_2$, $R_3$, $R_4$, and $R_5$ are each independently a hydrogen atom, a halogen atom, a C1-6 alkyl group, a hydroxy group, a C1-6 alkoxy group, a phenoxy group, a nitro group, or a cyano group.

**2.** The method according to claim 1, wherein the sodium base comprises at least one selected from the group consisting of sodium C1-6 alkoxides, sodium hydroxide, sodium hydride, and sodium hexamethyldisilazide.

**3.** The method according to claim 1 or 2, wherein

$X_1$ is a hydroxy group, and
$R_1$, $R_2$, $R_3$, $R_4$, and $R_5$ are each independently a hydrogen atom or a C1-6 alkyl group.

**4.** The method according to any one of claims 1 to 3, wherein said organic acid is at least one selected from the group consisting of o-cresol, m-cresol, p-cresol, phenol, and dibutylhydroxytoluene.

**5.** The method according to any one of claims 1 to 4, wherein said organic acid is an acid weaker than compound 1.

**6.** The method according to any one of claims 1 to 5, wherein a solubility at 25°C of a sodium salt of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 3 mmol/L or more.

**7.** The method according to any one of claims 1 to 6, wherein a solubility at 25°C of said organic acid in a mixed solvent of tetrahydrofuran, acetone, tert-butyl methyl ether, and water (a volume ratio of tetrahydrofuran, acetone, and tert-butyl methyl ether is 8:10:5, and a content of water is 3% by mass with respect to a mass of said mixed solvent) is 0.05 mol/L or more.

**8.** The method according to any one of claims 1 to 7, wherein a molar equivalent of the organic acid mixed in step (A-1) is 0.1 to 3 with respect to the sodium base.

**9.** The method according to any one of claims 1 to 8, further comprising step (A-3) below:
(A-3) obtaining compound 3 from the mixture obtained in step (A-1).

**10.** A method for producing 2-(4-cyclopropyl-2-fluoroanilino)-3,4-difluoro-5-[[3-fluoro-2-(methylsulfamoylamino)pyridin-4-yl]methyl]benzamide (hereinafter, also referred to as "compound 1"), the method comprising steps (B-1), (B-2), and (B-3) below:

(B-1) mixing (d), (e), and (f) below to obtain a mixture containing compound 1:

(d) 5-[(2-amino-3-fluoropyridin-4-yl)methyl]-2-(4-cyclopropyl-2-fluoroanilino)-3,4-difluorobenzamide (hereinafter, also referred to as "compound 2");
(e) N-methylsulfamoyl chloride; and
(f) an aqueous solution of an inorganic salt;

(B-2) removing an aqueous layer from the mixture obtained in step (B-1) by a separating operation; and
(B-3) mixing the mixture obtained in step (B-2) with a fourth solvent to remove said inorganic salt.

**11.** A method for producing a sodium salt of 2-(4-cyclopropyl-2-fluoroanilino)-3,4-difluoro-5-[[3-fluoro-2-(methylsulfamoylamino)pyridin-4-yl]methyl]benzamide (hereinafter, also referred to as "compound 3"), the method comprising step (C-1) below:
(C-1) producing 2-(4-cyclopropyl-2-fluoroanilino)-3,4-difluoro-5-[[3-fluoro-2-(methylsulfamoylamino)pyridin-4-yl]methyl]benzamide (compound 1) by the method according to claim 10.

**12.** A composition comprising a sodium salt of 2-(4-cyclopropyl-2-fluoroanilino)-3,4-difluoro-5-[[3-fluoro-2-(methylsulfamoylamino)pyridin-4-yl]methyl]benzamide (hereinafter, also referred to as "compound 3"), wherein a content of compound 3 is 98.0% by mass or more with respect to a mass of said composition.

**13.** A composition comprising a sodium salt of 2-(4-cyclopropyl-2-fluoroanilino)-3,4-difluoro-5-[[3-fluoro-2-(methylsulfamoylamino)pyridin-4-yl]methyl]benzamide (hereinafter, also referred to as "compound 3") and 2-(4-cyclopropyl-2-fluoroanilino)-3,4-difluoro-5-[[3-fluoro-2-(methylsulfamoylamino)pyridin-4-yl]methyl]benzoic acid (hereinafter, also referred to as "compound 4"),
wherein a peak area of compound 4 obtained by high-performance liquid chromatography (HPLC) analysis at 278 nm for said composition is 0.20% or less with respect to a peak area of compound 3 obtained by said HPLC analysis.

**14.** The composition according to claim 13, which is a pharmaceutical composition.

**15.** The method according to any one of claims 1 to 9 and 11, wherein a product with a high content of compound 3 can be obtained.

Fig.1

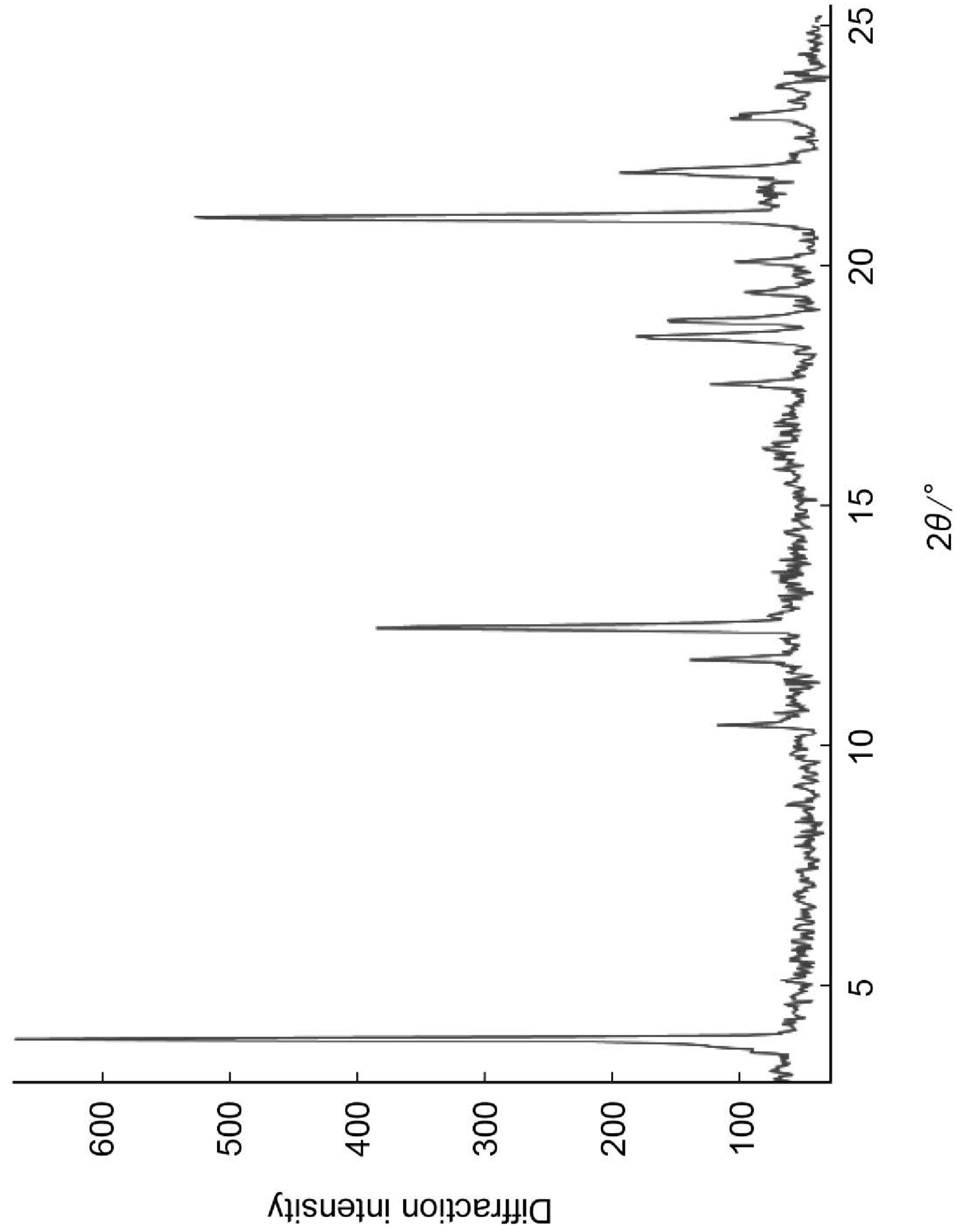

Fig.2

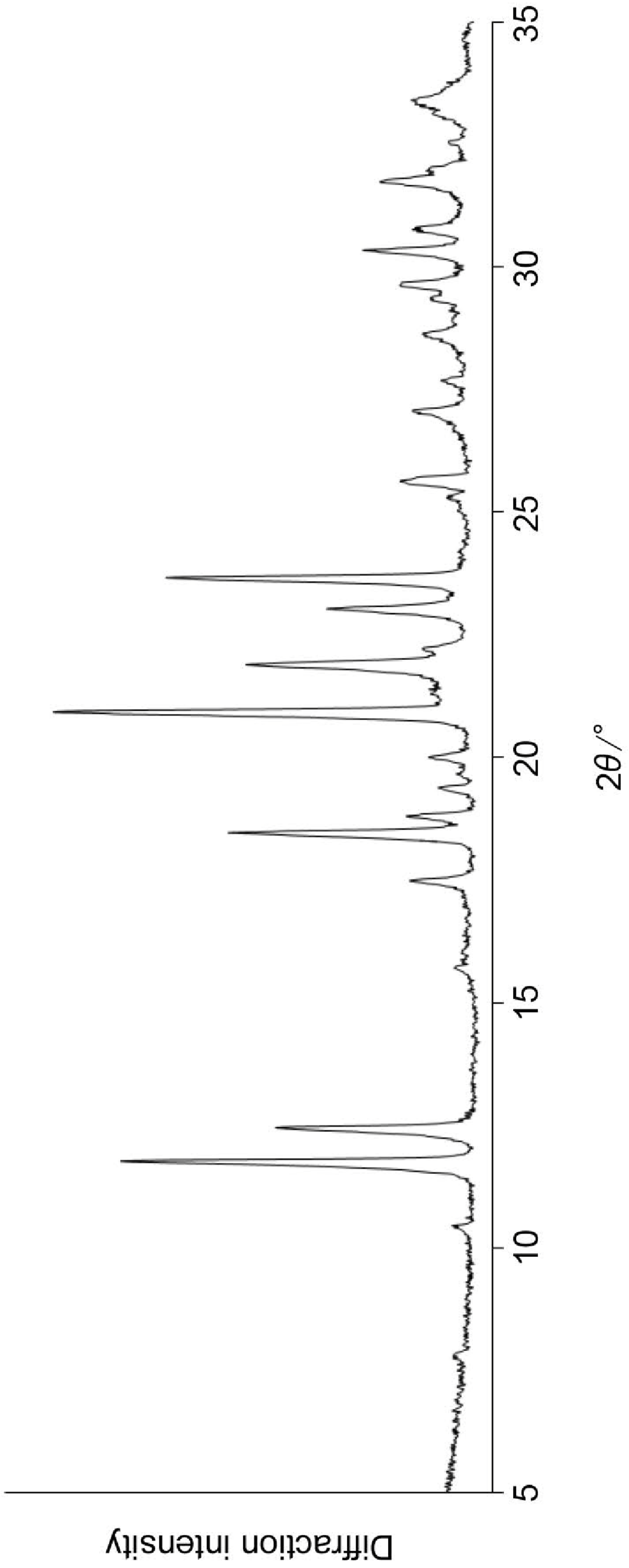

Fig.3

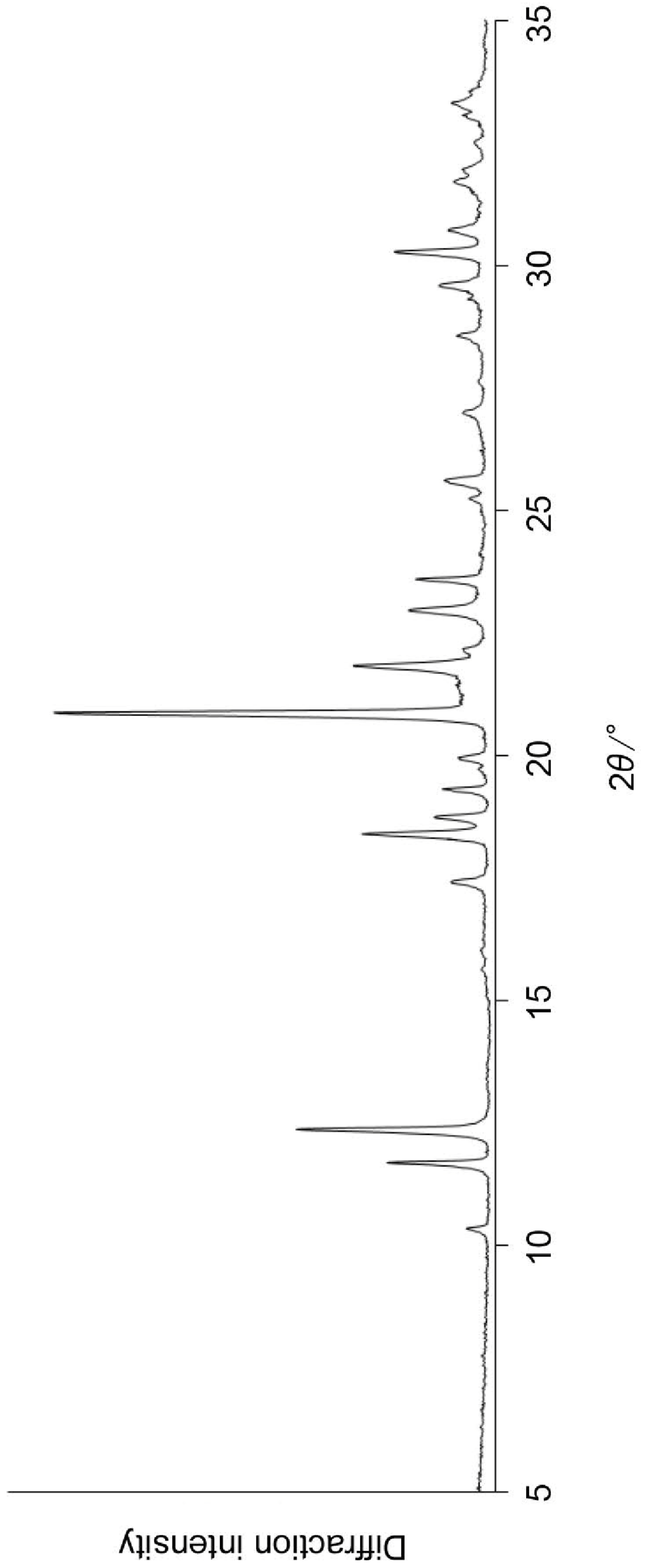

**INTERNATIONAL SEARCH REPORT**

International application No.
**PCT/JP2024/033634**

**A. CLASSIFICATION OF SUBJECT MATTER**

***C07D 213/76***(2006.01)i; ***A61K 31/44***(2006.01)i; ***A61P 35/00***(2006.01)i
FI: C07D213/76; A61P35/00; A61K31/44

According to International Patent Classification (IPC) or to both national classification and IPC

**B. FIELDS SEARCHED**

Minimum documentation searched (classification system followed by classification symbols)

C07D213/76; A61K31/44; A61P35/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2022/258612 A1 (F. HOFFMANN-LA ROCHE AG) 15 December 2022 (2022-12-15) claims pp. 22-23 | 12-14 |
| A | claims pp. 22-23 | 1-11, 15 |
| X | JP 2022-145416 A (CHUGAI SEIYAKU KABUSHIKI KAISHA) 04 October 2022 (2022-10-04) claims, paragraphs [0229]-[0231] | 12-14 |
| A | claims, paragraphs [0229]-[0231] | 1-11, 15 |
| X | JP 7022873 B1 (CHUGAI SEIYAKU KABUSHIKI KAISHA) 18 February 2022 (2022-02-18) claims, paragraphs [0244]-[0246] | 12-14 |
| A | claims, paragraphs [0244]-[0246] | 1-11, 15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 November 2024** | **03 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/033634**

| Patent document cited in search report | Publication date (day/month/year) | Patent family member(s) | Publication date (day/month/year) |
|---|---|---|---|
| WO 2022/258612 A1 | 15 December 2022 | JP 2023-177177 A | |
| | | JP 2023-177209 A | |
| | | JP 2023-177335 A | |
| | | US 2024/0139192 A1 | |
| | | EP 4351577 A1 | |
| | | AU 2022288118 A | |
| | | IL 307964 A | |
| | | CA 3222549 A1 | |
| | | KR 10-2024-0005899 A | |
| | | KR 10-2024-0008410 A | |
| | | CN 117642166 A | |
| | | BR 112023025916 A | |
| | | MX 2023014565 A | |
| | | TW 202313046 A | |
| JP 2022-145416 A | 04 October 2022 | JP 2023-11759 A | |
| | | WO 2022/018875 A1 | |
| | | KR 10-2022-0012195 A | |
| | | KR 10-2023-0098762 A | |
| JP 7022873 B1 | 18 February 2022 | US 2023/0270730 A1 claims, paragraphs [1194]-[1199] | |
| | | JP 2022-60295 A | |
| | | WO 2022/019329 A1 | |
| | | EP 4186506 A1 | |
| | | TW 202216140 A | |
| | | KR 10-2023-0003296 A | |
| | | CN 115803024 A | |
| | | BR 112023000883 A | |
| | | AU 2021311665 A | |
| | | CA 3186655 A | |
| | | IL 300051 A | |
| | | KR 10-2023-0122174 A | |
| | | PE 20231094 A | |
| | | CR 20230095 A | |
| | | ZA 202302083 B | |
| | | CL 2023000199 A | |
| | | MX 2023000857 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021149776 A
- WO 2023003014 A


### Non-patent literature cited in the description


- *Org. Biomol. Chem.*, 2009, vol. 7, 5103
- IUPAC Chemical Data Series. Pergamon Press, 1979
- Handbook of Biochemistry and Selected Data for Molecular Biology. 1970
- Handbook of Chemistry and Physics. CRC, 2004